# EUROPEAN PATENT APPLICATION

(11) **EP 4 339 194 A1**
(43) Date of publication of application: **20.03.2024**
(21) Application number: 22806854.0
(22) Date of filing: 13.05.2022
(51) Int. Cl.: C07D 403/04, A61K 31/40, A61K 31/415, A61P 11/00, A61P 9/00, A61P 25/00

(54) **ACID ADDITION SALT OF ROCK INHIBITOR, AND CRYSTAL FORM, COMPOSITION AND PHARMACEUTICAL USE THEREOF**

(30) Priority: 14.05.2021 CN 202110533087; 07.05.2022 CN 202210500178
(71) Applicant: Wuhan LL Science and Technology Development Co., Ltd., Wuhan, Hubei 430075 (CN)
(72) Inventor: WANG, Liang, Wuhan, Hubei 430075 (CN); LOU, Jun, Wuhan, Hubei 430075 (CN); YUAN, Yi, Wuhan, Hubei 430075 (CN); GUO, Xiaodan, Wuhan, Hubei 430075 (CN); CHEN, Yongkai, Wuhan, Hubei 430075 (CN); ZHANG, Yihan, Wuhan, Hubei 430075 (CN); HONG, Huayun, Wuhan, Hubei 430075 (CN); PENG, Wei, Wuhan, Hubei 430075 (CN); WANG, Chaodong, Wuhan, Hubei 430075 (CN)
(74) Representative: Gille Hrabal Partnerschaftsgesellschaft mbB Patentanwälte
(86) International application number: PCT/CN2022/092622
(87) International publication number: WO 2022/237892

(57) **Abstract**

An acid addition salt of a ROCK inhibitor, and a crystal form, a composition and the pharmaceutical use thereof. The acid addition salt is an acid addition salt of compound A and any one of the following acids: hydrochloric acid, p-toluenesulfonic acid, benzenesulfonic acid, maleic acid, tartaric acid, oxalic acid, fumaric acid, sulfuric acid, methanesulfonic acid, phosphoric acid, succinic acid or citric acid (A). The acid addition salt of compound A and the crystal form thereof have the characteristics of high solubility, good stability, high purity, few impurities and high bioequivalence, and are beneficial for the storage, quality control and druggability of drugs.

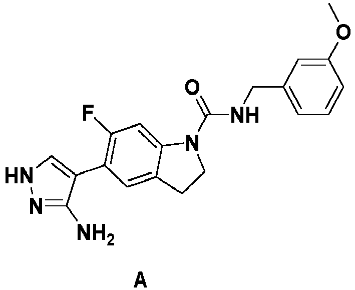

## Description

The present application claims priority to prior applications of the Patent Application for Invention with the application No. 202110533087.9 and entitled "ACID ADDITION SALT OF ROCK INHIBITOR, AND CRYSTAL FORM, COMPOSITION AND PHARMACEUTICAL USE THEREOF" filed with China National Intellectual Property Administration on May 14, 2021 and the Patent Application for Invention with the application No. 202210500178.7 and entitled "ACID ADDITION SALT OF ROCK INHIBITOR, AND CRYSTAL FORM, COMPOSITION AND PHARMACEUTICAL USE THEREOF" filed with China National Intellectual Property Administration on May 7, 2022, which are incorporated herein by reference in their entirety.

### TECHNICAL FIELD

The present disclosure belongs to the field of pharmaceutical chemistry, and particularly relates to an acid addition salt of a ROCK inhibitor, a crystal form of the salt, a composition, and pharmaceutical use.

### BACKGROUND

Idiopathic interstitial pulmonary fibrosis (IPF) is a chronic and diffuse pulmonary interstitial disease with unknown cause and its pathological change is common interstitial pneumonia. It mainly shows manifestation of common interstitial pneumonia according to histopathology and imageology examinations. The disease condition progresses irreversibly due to the complex pathogenesis of the disease, and early diagnosis is difficult. The survival rate of patients diagnosed with the disease is remarkably decreased over time, with a 3-year survival rate of 50% and a 5-year survival rate of only 20%, which is lower than that of most cancers (such as leukemia, breast cancer, colon cancer, uterine tumor and renal cancer), and therefore the disease is called "cancer that is not cancer". At present, there is no definitely significantly effective therapeutic drug for IPF. According to the results of randomized control clinical trials in recent years and the actual clinical conditions in China, drugs such as pirfenidone and nintedanib can be used as appropriate. Only IPF patients with mild to moderate pulmonary dysfunction are recommended to be treated by nintedanib, while whether IPF patients with severe pulmonary dysfunction can benefit from treatment by nintedanib and the course of treatment still need to be further discussed.

Rho GTPase was discovered in 1985, and it belongs to the Ras superfamily and has 25% homology to Ras. At present, Rho GTPase members found in mammalian tissue cells mainly include Rho (A, B, C), Rac (1, 2, 3), Cdc42 (Cdc42Hs/G25K, TC10, Tcl), Rho D, Rho G, Chp (1, 2), Rnd (Rho E/Rnd3, Rnd1/Rho6, Rnd2/Rho7), Rho H/TTF, Rif, Wrch1 and Rho BTB (1, 2). Among them, Rho (A, B, C) is one of the most important members of the Rho GTPase family. Rho-associated protein kinase (ROCK), also called Rho-associated kinase, is a serine/threonine protein kinase with a molecular mass of about 160 kD, and is a Rho downstream effector molecule which is the most functionally studied downstream target effector molecule of Rho. ROCK includes ROCK1 (ROKβ, p160-ROCK) and ROCK2 (ROKα) subtypes. The amino acid sequences of the two subtypes are 65% identical, with a high degree of similarity (92% identity) in the kinase domain. ROCK is distributed throughout the body, and in comparison, ROCK1 is more highly expressed in non-neural tissues (blood, small intestine, thymus, etc.), while ROCK2 is more highly expressed in brain, heart and colon.

ROCK is involved in occurrence of various cardiovascular and cerebrovascular diseases, including hypertension, atherosclerosis, ischemic stroke, heart disease, diabetic nephropathy, ocular diseases, tumors, nerve injury diseases, radiation damage, autoimmune diseases and the like. For example, the Rho/ROCK signaling pathway is involved in NAD(P)H oxidase activation and induces oxidative stress, inducing cardiac microvascular damage and C-reactive protein-induced atherothrombosis; high glucose can activate the Rho/ROCK pathway, induce the expression of visfatin and type I procollagen in cardioblasts, and cause the hyperproliferation of cardioblast and thus induce diabetic cardiomyopathy; activation of the Rho/ROCK signaling pathway can regulate NF-κB signaling pathway, up-regulate inflammatory genes and induce the occurrence of diabetic nephropathy; Rho/ROCK signaling pathway changes biofilm permeability and affects metastasis of cancer cells; in the case of spinal cord injury, Rho is activated, inducing atrophy of growth cones and thereby causing axonal regeneration disorder and simultaneously inducing inhibition against neuron growth by chondroitin sulfate proteoglycan.

In addition, the Rho/ROCK signaling pathway is involved in the occurrence and progression of fibrosis diseases. Activation of Rho/ROCK signaling pathway can increase the level of ischemic myocardial fibrosis, and heart tissues of an acute myocardial fibrosis rat show significantly increased Rho and ROCK expression. Activation of Rho/ROCK signaling pathway can induce phosphorylation of actin, initiating cellular fibrosis. Both *in vivo* and *in vitro* experimental results demonstrate that cardiopulmonary physiological and pathological damage caused by exposure to radiation over a period of time is associated with fibrosis induced with the involvement of Rho/ROCK pathway. Formation of endothelial adhesion fibronectin and focal adhesion, decreased endothelial cell migration and endothelial dysfunction due to ionizing radiation are associated with actin scaffold reorganization and stress fiber formation induced by activation of Rho/ROCK signaling pathway.

With respect to lung injury by IPF, primarily alveolar epithelial cells (ACEs) are the targets, and the death of ACEs triggers wound healing responses including innate immune activation, vascular leakage and extravascular coagulation, fibroblast recruitment, proliferation and activation, synthesis and cross-linking of extracellular matrix, alveolar collapse and epithelial regeneration. ROCK signals can fundamentally regulate the activities of these cells involved in the healing response, particularly those of epithelial cells, endothelial cells, and fibroblasts. The key role of ROCK in these responses further suggests the potential of ROCK inhibitors for the treatment of pulmonary fibrosis.

Currently, no drugs that treat numerous disorders including fibrosis through inhibition of ROCK are available on the market. The development of new medicaments requires careful optimization of the chemical and biological properties of the lead compounds. Further, the compounds must have desired pharmacokinetic and pharmacodynamic characteristics. This laborious development process typically requires extensive experimentation. In many cases, the process of determining the optimal compound often requires the preparation of thousands of structurally similar compounds. Therefore, the development of a novel backbone compound having an inhibitory effect on ROCK1 and/or ROCK2 kinases by improving a ROCK kinase inhibitor is of positive significance for the treatment of the diseases described above. Meanwhile, the development of pharmaceutical solid forms of these compounds suitable for pharmaceutical preparations, for example, solid forms with improved stability, hygroscopicity and/or efficacy, has become an urgent technical problem to be solved so as to achieve favorable results in the pharmaceutical and therapeutic stages.

### SUMMARY

The present disclosure provides a salt of compound A, wherein compound A is shown as the following structure: 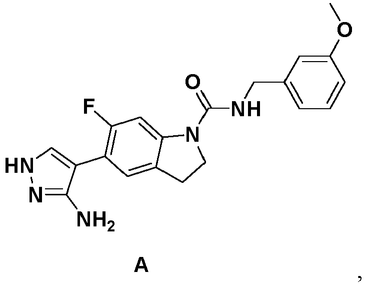 the salt is an acid addition salt, wherein, for example, the salt is an acid addition salt of compound A with any one of the following acids: hydrochloric acid, *p*-toluenesulfonic acid, benzenesulfonic acid, maleic acid, tartaric acid (including L-tartaric acid or R-tartaric acid), oxalic acid, fumaric acid, sulfuric acid, methanesulfonic acid, phosphoric acid, succinic acid, or citric acid, preferably hydrochloric acid, *p*-toluenesulfonic acid, benzenesulfonic acid, maleic acid, tartaric acid, oxalic acid, and fumaric acid.

Preferably, the acid addition salt is a hydrochloride salt of compound A, a *p*-toluenesulfonate salt of compound A, a benzenesulfonate salt of compound A, a maleate salt of compound A, a tartrate salt of compound A, an oxalate salt of compound A, or a fumarate salt of compound A.

According to a technical solution of the present disclosure, in the salt of compound A, compound A and the acid are in a molar ratio of 5:1-1:5, e.g., 3:1, 2:1, 1:1, 1:1.5, 1:2, 1:2.5, or 1:3.

According to a technical solution of the present disclosure, the salt of compound A may be in an amorphous or crystal form.

For example, the hydrochloride salt of compound A may be in an amorphous or crystal form of the hydrochloride salt of compound A, the *p*-toluenesulfonate salt of compound A may be in an amorphous or crystal form of the *p*-toluenesulfonate salt of compound A, the benzenesulfonate salt of compound A may be in an amorphous or crystal form of the benzenesulfonate salt of compound A, the maleate salt of compound A may be in an amorphous or crystal form of the maleate salt of compound A, the tartrate salt of compound A may be in an amorphous or crystal form of the tartrate salt of compound A, the oxalate salt of compound A may be in an amorphous or crystal form of the oxalate salt of compound A, and the fumarate salt of compound A may be in an amorphous or crystal form of the fumarate salt of compound A.

Preferably, the acid addition salt of compound A is in a crystal form selected from the crystal form of the hydrochloride salt of compound A, the crystal form of the *p*-toluenesulfonate salt of compound A, the crystal form of the benzenesulfonate salt of compound A, the crystal form of the maleate salt of compound A, the crystal form of the tartrate salt of compound A, the crystal form of the oxalate salt of compound A, and the crystal form of the fumarate salt of compound A.

According to a technical solution of the present disclosure, the hydrochloride salt of compound A is in a crystal form, which is named as a hydrochloride crystal form I. The hydrochloride crystal form I has characteristic peaks at 2θ angles of 5.93° ± 0.20°, 14.92° ± 0.20°, and 24.07° ± 0.20° by X-ray powder diffraction using Cu-Kα radiation.

Preferably, the hydrochloride crystal form I has characteristic peaks at 2θ angles of 5.93° ± 0.20°, 11.96° ± 0.20°, 14.92° ± 0.20°, 17.98° ± 0.20°, 24.07° ± 0.20°, 26.61° ± 0.20°, and 27.18° ± 0.20° by X-ray powder diffraction using Cu-Kα radiation.

Further preferably, the hydrochloride crystal form I has characteristic peaks at 2θ angles of 5.93° ± 0.20°, 11.96° ± 0.20°, 12.56° ± 0.20°, 14.92° ± 0.20°, 17.98° ± 0.20°, 18.96° ± 0.20°, 21.02° ± 0.20°, 24.07° ± 0.20°, 25.53° ± 0.20°, 26.61° ± 0.20°, 27.18° ± 0.20°, and 31.66° ± 0.20° by X-ray powder diffraction using Cu-Kα radiation.

Preferably, the hydrochloride crystal form I has characteristic peaks at 2θ angles by X-ray powder diffraction as shown in Table 5', with a tolerance range of ± 0.2°.

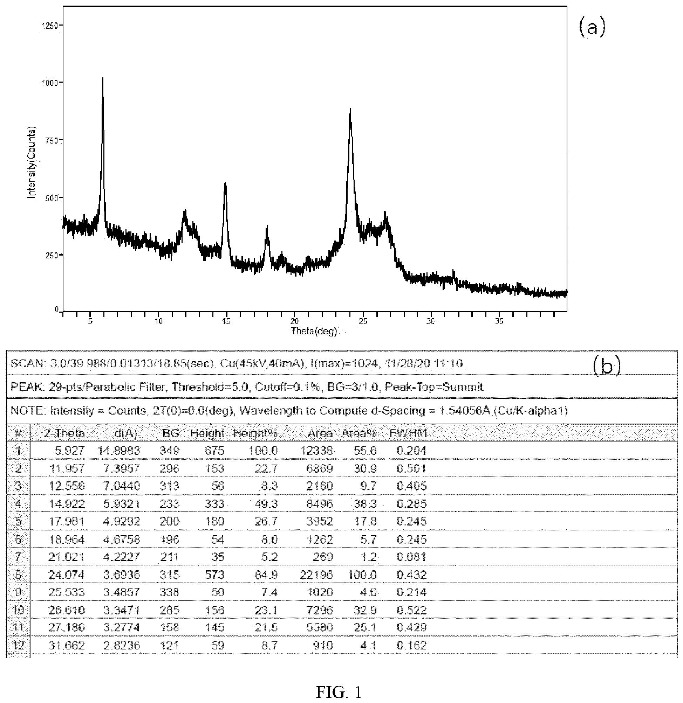

Preferably, the hydrochloride crystal form I has characteristic peaks at 2θ angles by X-ray powder diffraction as shown in FIG. 1(b), with a tolerance range of ± 0.20°.

According to a technical solution of the present disclosure, the hydrochloride crystal form I has an XRPD pattern substantially as shown in FIG. 1(a).

According to a technical solution of the present disclosure, in the hydrochloride crystal form I, compound A and the hydrochloric acid are in a molar ratio of 1:1.

According to a technical solution of the present disclosure, the hydrochloride crystal form I is a hydrate, preferably a monohydrate.

According to a technical solution of the present disclosure, the hydrochloride crystal form I has a weight loss of about 5.4% at room temperature to about 110 °C.

In some embodiments, the hydrochloride crystal form I has a weight loss of 5.4% ± 2% at room temperature to 110 °C ± 3 °C.

According to a technical solution of the present disclosure, the hydrochloride crystal form I has a broad endothermic peak with a peak temperature of about 97 °C, preferably with a peak temperature of 97 °C ± 5 °C, e.g., 97 °C ± 2 °C.

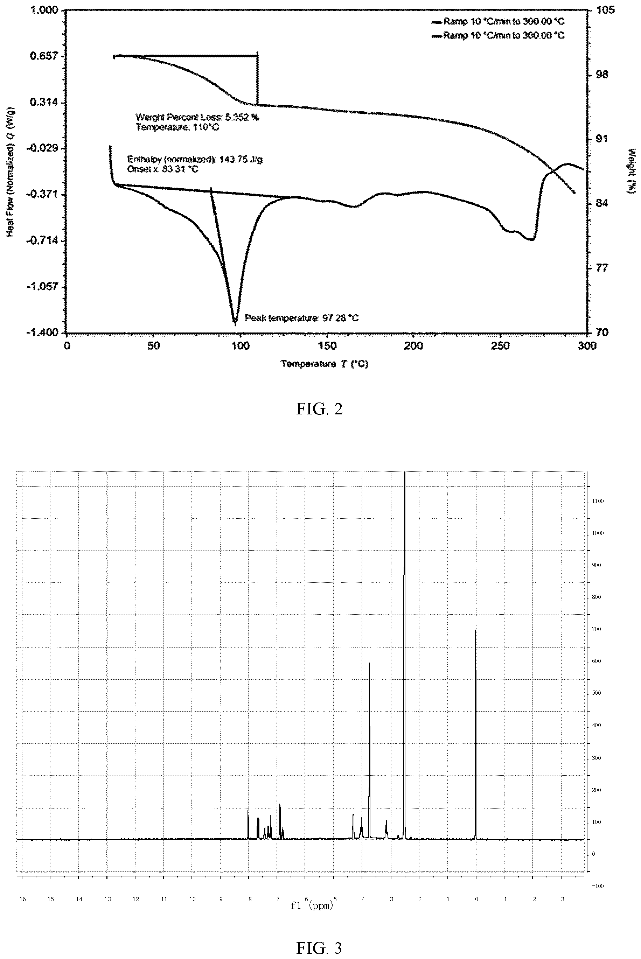

According to a technical solution of the present disclosure, the hydrochloride crystal form I has DSC and TGA patterns substantially as shown in FIG. 2.

According to a technical solution of the present disclosure, the hydrochloride crystal form I comprises no organic solvent.

According to a technical solution of the present disclosure, the *p*-toluenesulfonate salt of compound A is in a crystal form, which is named as a *p*-toluenesulfonate crystal form I. The *p*-toluenesulfonate crystal form I has characteristic peaks at 2θ angles of 7.55° ± 0.20°, 8.61° ± 0.20°, 14.75° ± 0.20°, 15.99° ± 0.20°, and 23.38° ± 0.20° by X-ray powder diffraction using Cu-Kα radiation.

Preferably, the *p*-toluenesulfonate crystal form I has characteristic peaks at 2θ angles of 7.55° ± 0.20°, 8.61° ± 0.20°, 14.75° ± 0.20 °, 15.99° ± 0.20°, 19.64° ± 0.20°, 19.91° ± 0.20°, 23.38° ± 0.20°, 24.02° ± 0.20°, and 24.60° ± 0.20° by X-ray powder diffraction using Cu-Kα radiation.

Further preferably, the *p*-toluenesulfonate crystal form I has characteristic peaks at 2θ angles of 5.49° ± 0.20°, 7.55° ± 0.20°, 8.61° ± 0.20°, 9.14° ± 0.20°, 10.05° ± 0.20°, 14.39° ± 0.20°, 14.75° ± 0.20°, 15.99° ± 0.20°, 19.64° ± 0.20°, 19.91° ± 0.20°, 20.67° ± 0.20°, 23.38° ± 0.20°, 24.02° ± 0.20°, and 24.60° ± 0.20° by X-ray powder diffraction using Cu-Kα radiation.

Preferably, the *p*-toluenesulfonate crystal form I has characteristic peaks at 2θ angles by X-ray powder diffraction as shown in Table 8', with a tolerance range of ± 0.2°.

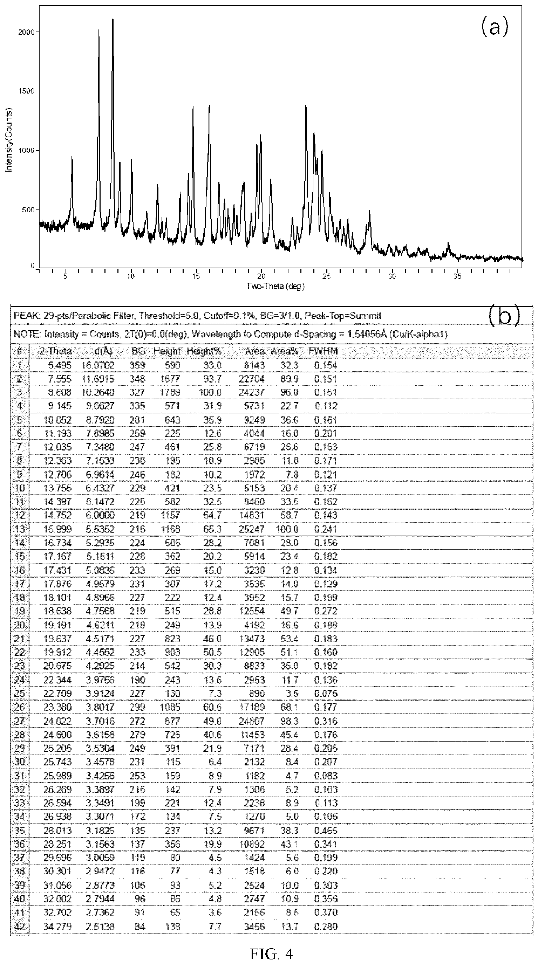

Preferably, the *p*-toluenesulfonate crystal form I has characteristic peaks at 2θ angles by X-ray powder diffraction as shown in FIG. 4(b), with a tolerance range of ± 0.20°.

According to a technical solution of the present disclosure, the *p*-toluenesulfonate crystal form I has an XRPD pattern substantially as shown in FIG. 4(a).

According to a technical solution of the present disclosure, in the *p*-toluenesulfonate crystal form I, compound A and *p*-toluenesulfonic acid are in a molar ratio of 1:1.

According to a technical solution of the present disclosure, the *p*-toluenesulfonate crystal form I is a hydrate, preferably a monohydrate.

According to a technical solution of the present disclosure, the *p*-toluenesulfonate crystal form I has a weight loss of about 2.5% at room temperature to about 105 °C.

In some embodiments, the *p*-toluenesulfonate crystal form I has a weight loss of 2.5% ± 2% at room temperature to 105 °C ± 3 °C.

According to a technical solution of the present disclosure, the *p*-toluenesulfonate crystal form I has two absorption peaks, wherein the first absorption peak has a peak temperature of about 89 °C (the peak shape is shown as a broad endothermic peak), and the second absorption peak has a peak temperature of about 127 °C.

In some embodiments, the *p*-toluenesulfonate crystal form I has two absorption peaks, wherein the first absorption peak has a peak temperature of 89 °C ± 5 °C, e.g., 89 °C ± 2 °C; the second absorption peak has a peak temperature of 127 °C ± 5 °C, e.g., 127 °C ± 2 °C.

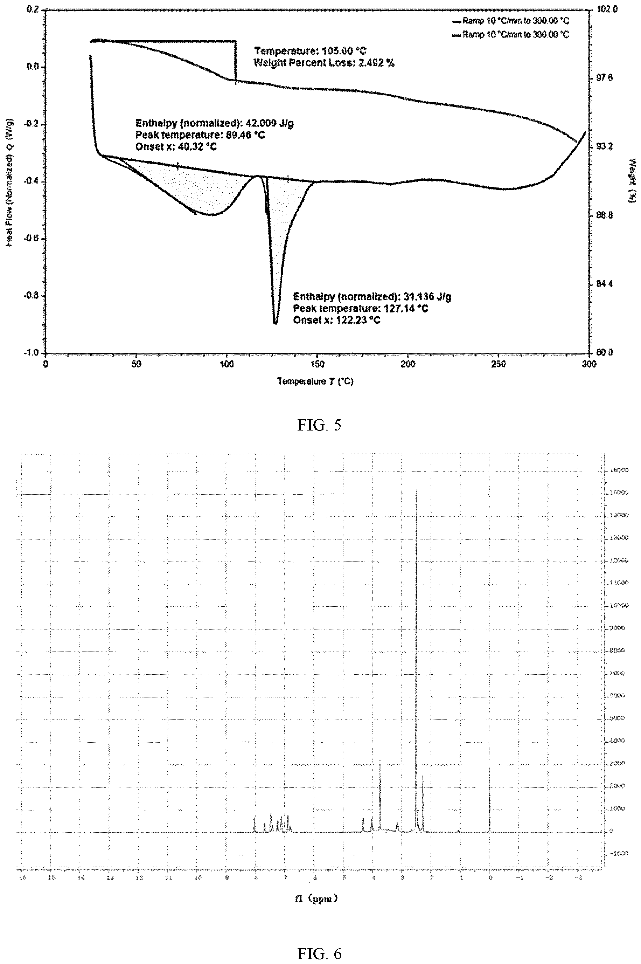

According to a technical solution of the present disclosure, the *p*-toluenesulfonate crystal form I has DSC and TGA patterns substantially as shown in FIG. 5.

According to a technical solution of the present disclosure, the *p*-toluenesulfonate crystal form I comprises an organic solvent. Preferably, the organic solvent has a content of less than 1%, further preferably, less than 0.6%. The organic solvent is selected from one, two or more of the following organic solvents: ethanol (EtOH), methyl *tert*-butyl ether (MTBE), ethyl acetate (EA), acetonitrile (ACN), dichloromethane (DCM), and the like. Illustratively, the *p*-toluenesulfonate crystal form I comprises 0.5% ethanol (EtOH) and 0.1% methyl *tert*-butyl ether (MTBE).

According to a technical solution of the present disclosure, the benzenesulfonate salt of compound A is in a crystal form, which is named as a benzenesulfonate crystal form I. The benzenesulfonate crystal form I has characteristic peaks at 2θ angles of 7.96° ± 0.20°, 9.00° ± 0.20°, 15.80° ± 0.20°, 20.49° ± 0.20°, and 24.61° ± 0.20° by X-ray powder diffraction using Cu-Kα radiation.

Preferably, the benzenesulfonate crystal form I has characteristic peaks at 2θ angles of 7.96° ± 0.20°, 9.00° ± 0.20°, 15.28° ± 0.20°, 15.80° ± 0.20°, 19.97° ± 0.20°, 20.49° ± 0.20°, and 24.61° ± 0.20° by X-ray powder diffraction using Cu-Kα radiation.

Further preferably, the benzenesulfonate crystal form I has characteristic peaks at 2θ angles of 7.96° ± 0.20°, 9.00° ± 0.20°, 9.79° ± 0.20°, 10.30° ± 0.20°, 14.48° ± 0.20°, 15.28° ± 0.20°, 15.80° ± 0.20°, 17.09° ± 0.20°, 17.29° ± 0.20°, 19.24° ± 0.20°, 19.97° ± 0.20°, 20.49° ± 0.20°, 23.29° ± 0.20°, 24.61° ± 0.20°, and 25.24° ± 0.20° by X-ray powder diffraction using Cu-Kα radiation.

Preferably, the benzenesulfonate crystal form I has characteristic peaks at 2θ angles by X-ray powder diffraction as shown in Table 10', with a tolerance range of ± 0.2°.

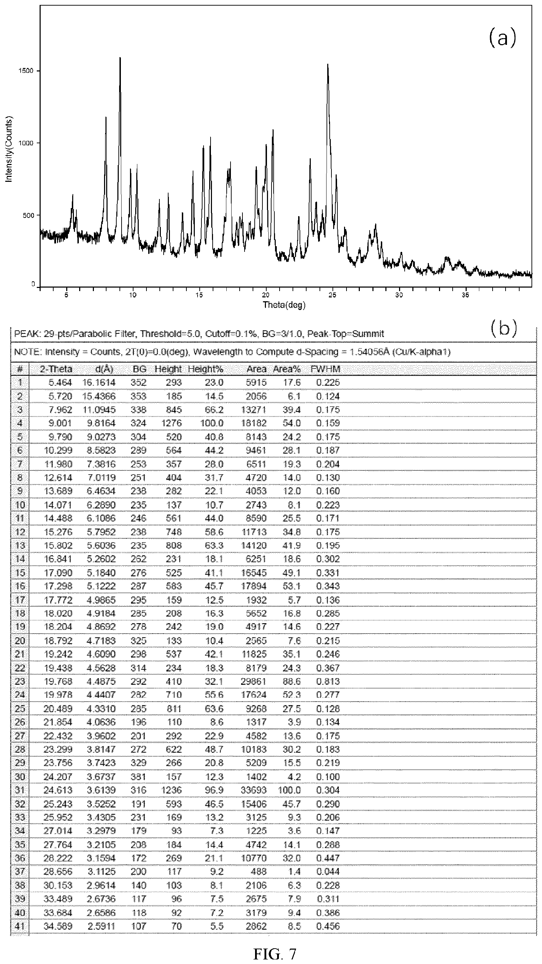

Preferably, the benzenesulfonate crystal form I has characteristic peaks at 2θ angles by X-ray powder diffraction as shown in FIG. 7(b), with a tolerance range of ± 0.20°.

According to a technical solution of the present disclosure, the benzenesulfonate crystal form I has an XRPD pattern substantially as shown in FIG. 7(a).

According to a technical solution of the present disclosure, in the benzenesulfonate crystal form I, compound A and benzenesulfonic acid are in a molar ratio of 1:1.

According to a technical solution of the present disclosure, the benzenesulfonate crystal form I is a hydrate, preferably a monohydrate.

According to a technical solution of the present disclosure, the benzenesulfonate crystal form I has a weight loss of about 3.0% at room temperature to about 117 °C.

In some embodiments, the benzenesulfonate crystal form I has a weight loss of 3.0% ± 2% at room temperature to 117 °C ± 3 °C.

According to a technical solution of the present disclosure, the benzenesulfonate crystal form I has a broad overlapping endothermic peak with a peak temperature of about 114 °C.

In some embodiments, the benzenesulfonate crystal form I has a broad overlapping endothermic peak with a peak temperature of 114°C ± 5 °C, e.g., 114 °C ± 2 °C.

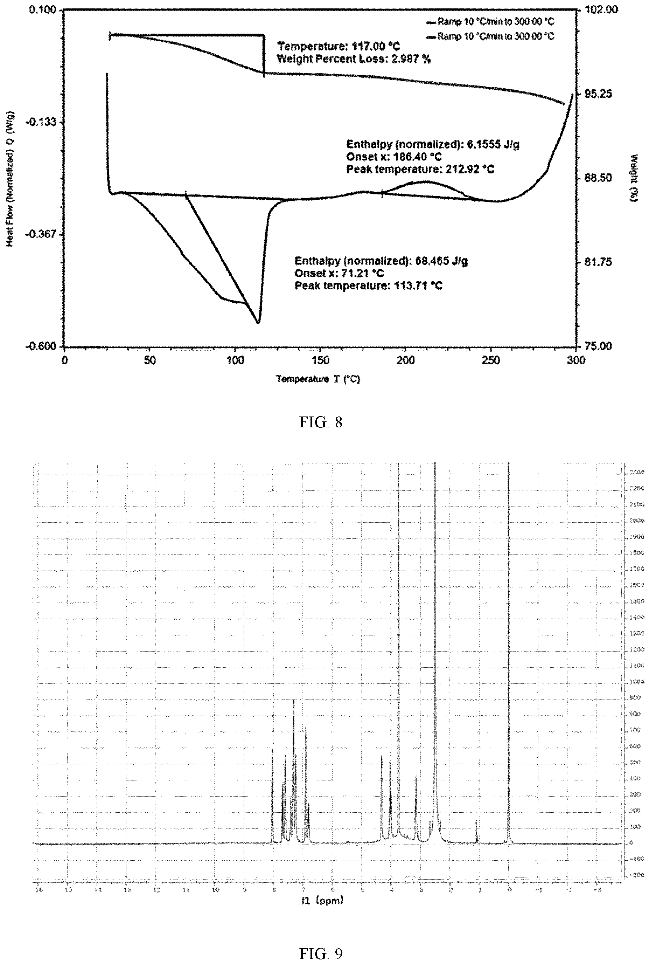

According to a technical solution of the present disclosure, the benzenesulfonate crystal form I has DSC and TGA patterns substantially as shown in FIG. 8.

According to a technical solution of the present disclosure, the benzenesulfonate crystal form I comprises an organic solvent. Preferably, the organic solvent has a content of less than 1%, further preferably, less than 0.6%. The organic solvent is selected from one, two or more of the organic solvents shown below: ethanol (EtOH), methyl *tert*-butyl ether (MTBE), ethyl acetate (EA), acetonitrile (ACN), dichloromethane (DCM), and the like. Illustratively, the benzenesulfonate crystal form I comprises 0.4% ethanol (EtOH) and 0.2% methyl *tert*-butyl ether (MTBE).

According to a technical solution of the present disclosure, the maleate salt of compound A is in a crystal form, which is named as a maleate crystal form I. The maleate crystal form I has characteristic peaks at 2θ angles of 4.22° ± 0.20°, 7.29° ± 0.20°, 16.13° ± 0.20°, 17.19° ± 0.20°, and 26.07° ± 0.20° by X-ray powder diffraction using Cu-Kα radiation.

Preferably, the maleate crystal form I has characteristic peaks at 2θ angles of 4.22° ± 0.20°, 7.29° ± 0.20°, 12.25° ± 0.20°, 14.68° ± 0.20°, 15.34° ± 0.20°, 16.13° ± 0.20°, 17.19° ± 0.20°, 19.21° ± 0.20°, 22.59° ± 0.20°, 26.07° ± 0.20° by X-ray powder diffraction using Cu-Kα radiation.

Preferably, the maleate crystal form I has characteristic peaks at 2θ angles by X-ray powder diffraction as shown in Table 12', with a tolerance range of ± 0.2°.

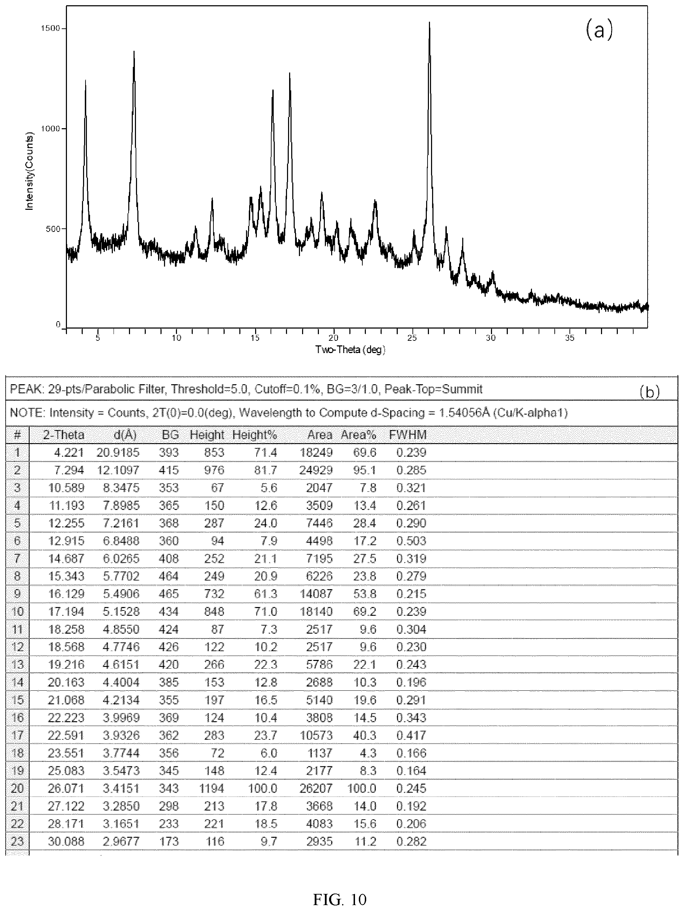

Preferably, the maleate crystal form I has characteristic peaks at 2θ angles by X-ray powder diffraction as shown in FIG. 10(b), with a tolerance range of ± 0.20°.

According to a technical solution of the present disclosure, the maleate crystal form I has an XRPD pattern substantially as shown in FIG. 10(a).

According to a technical solution of the present disclosure, in the maleate crystal form I, compound A and the maleic acid are in a molar ratio of 1:1.

According to a technical solution of the present disclosure, the maleate crystal form I has a weight loss of about 8.4% at room temperature to about 100 °C.

In some embodiments, the maleate crystal form I has a weight loss of 8.4% ± 2% at room temperature to 100 °C ± 3 °C.

According to a technical solution of the present disclosure, the maleate crystal form I has a broad overlapping endothermic peak with a peak temperature of about 83 °C.

In some embodiments, the maleate crystal form I has a broad overlapping endothermic peak with a peak temperature of 83 °C ± 5 °C, e.g., 83 °C ± 2 °C.

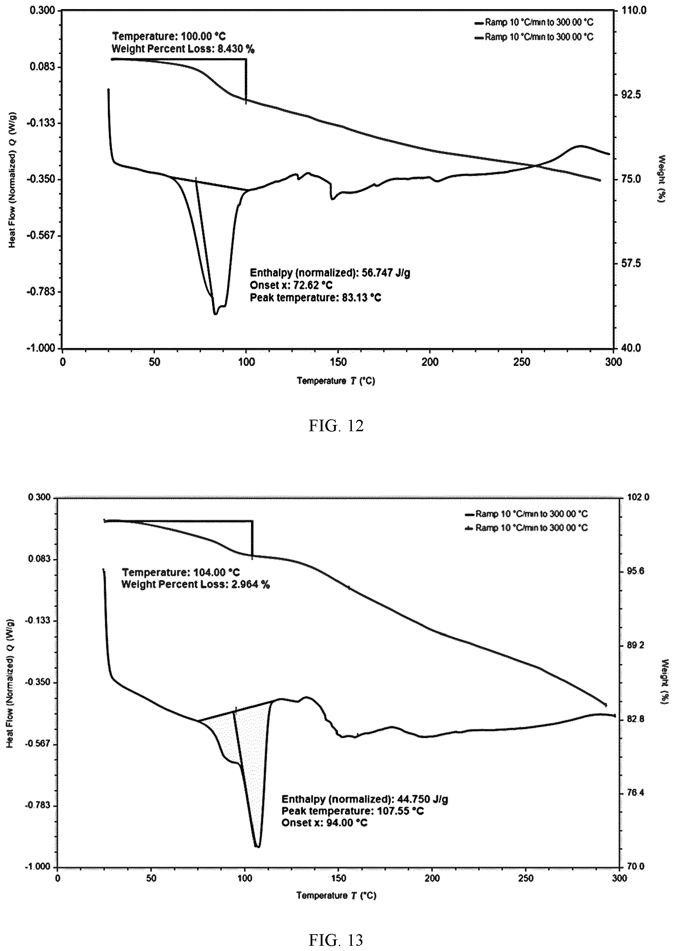

According to a technical solution of the present disclosure, the maleate crystal form I has DSC and TGA patterns substantially as shown in FIG. 12.

According to a technical solution of the present disclosure, the maleate crystal form I comprises an organic solvent. Preferably, the organic solvent has a content of less than 20%, further preferably, less than 15%, illustratively 14%, 13%, 12%, or 10%. The organic solvent is selected from one, two or more of the organic solvents shown below: ethanol (EtOH), methyl *tert*-butyl ether (MTBE), ethyl acetate (EA), acetonitrile (ACN), dichloromethane (DCM), and the like, preferably ethyl acetate (EA). Illustratively, the maleate crystal form I comprises 13% ethyl acetate (EA).

In some embodiments, the maleate crystal form I is a solvate, wherein the solvent has a content of less than 20%, preferably less than 15%, illustratively 14%, 13%, 12%, or 10%. Preferably, the maleate crystal form I is an ethyl acetate solvate.

According to a technical solution of the present disclosure, the maleate salt of compound A is in a crystal form, which is named as a maleate crystal form II. The maleate crystal form II has characteristic peaks at 2θ angles of 7.99° ± 0.20° and 20.17° ± 0.20° by X-ray powder diffraction using Cu-Kα radiation.

Preferably, the maleate crystal form II has characteristic peaks at 2θ angles of 3.96° ± 0.20°, 7.99° ± 0.20°, 20.17° ± 0.20°, 24.23° ± 0.20°, 28.31° ± 0.20° by X-ray powder diffraction using Cu-Kα radiation.

Further preferably, the maleate crystal form II has characteristic peaks at 2θ angles of 3.96° ± 0.20°, 7.99° ± 0.20°, 9.25° ± 0.20°, 11.19° ± 0.20°, 13.25° ± 0.20°, 20.17° ± 0.20°, 23.85° ± 0.20°, 24.23° ± 0.20°, 27.47° ± 0.20°, and 28.31° ± 0.20° by X-ray powder diffraction using Cu-Kα radiation.

Preferably, the maleate crystal form II has characteristic peaks at 2θ angles by X-ray powder diffraction as shown in Table 12", with a tolerance range of ± 0.2°.

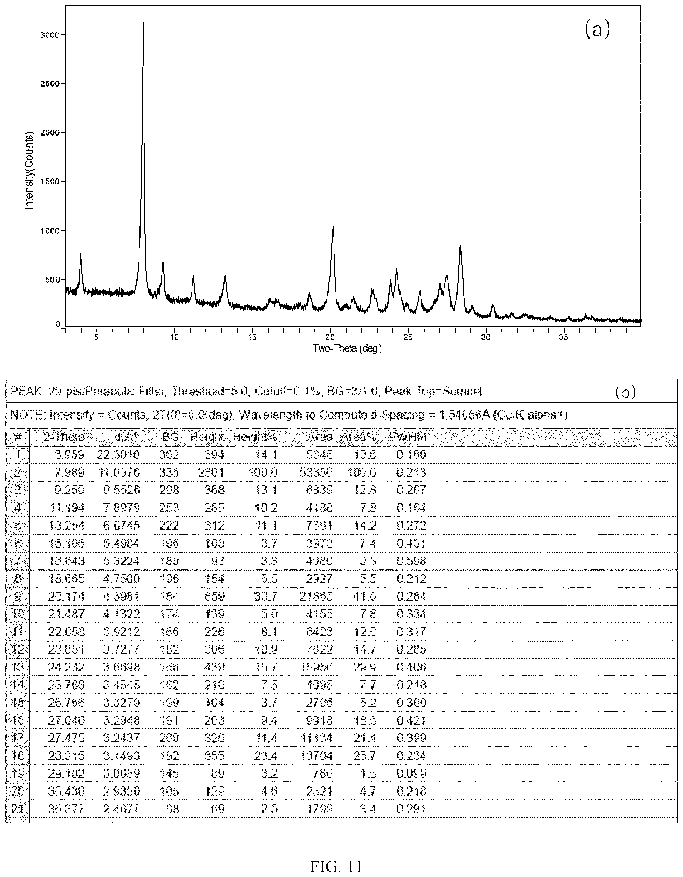

Preferably, the maleate crystal form II has characteristic peaks at 2θ angles by X-ray powder diffraction as shown in FIG. 11(b), with a tolerance range of ± 0.20°.

According to a technical solution of the present disclosure, the maleate crystal form II has an XRPD pattern substantially as shown in FIG. 11(a).

According to a technical solution of the present disclosure, in the maleate crystal form II, compound A and the maleic acid are in a molar ratio of 1:1.

According to a technical solution of the present disclosure, the maleate crystal form II has a weight loss of about 3.0% at room temperature to about 104 °C.

In some embodiments, the maleate crystal form II has a weight loss of 3.0% ± 2% at room temperature to 104 °C ± 3 °C.

According to a technical solution of the present disclosure, the maleate crystal form II has a broad overlapping endothermic peak with a peak temperature of about 108 °C.

In some embodiments, the maleate crystal form II has a broad overlapping endothermic peak with a peak temperature of 108 °C ± 5 °C, e.g., 108 °C ± 2 °C.

According to a technical solution of the present disclosure, the maleate crystal form II has DSC and TGA patterns substantially as shown in FIG. 13.

According to a technical solution of the present disclosure, the maleate crystal form II contains an organic solvent. Preferably, the organic solvent has a content of less than 20%, further preferably, less than 15%, illustratively 14%, 13%, 12%, or 10%. The organic solvent is selected from one, two or more of the organic solvents shown below: ethanol (EtOH), methyl *tert*-butyl ether (MTBE), ethyl acetate (EA), acetonitrile (ACN), dichloromethane (DCM), methanol (MeOH), and the like, e.g., methyl *tert*-butyl ether (MTBE). Illustratively, the maleate crystal form II comprises 2% MTBE.

In some embodiments, the maleate crystal form II is a solvate and/or hydrate, wherein the solvent has a content of less than 20%, preferably less than 15%, illustratively 14%, 13%, 12%, or 10%. Preferably, the maleate crystal form II is a methyl *tert*-butyl ether (MTBE) solvate and/or hydrate. According to a technical solution of the present disclosure, the oxalate salt of compound A is in a crystal form, which is named as an oxalate crystal form I. The oxalate crystal form I has characteristic peaks at 2θ angles of 5.26° ± 0.20°, 12.24° ± 0.20°, and 25.75° ± 0.20° by X-ray powder diffraction using Cu-Kα radiation.

Preferably, the oxalate crystal form I has characteristic peaks at 2θ angles of 4.94° ± 0.20°, 5.26° ± 0.20°, 7.25° ± 0.20°, 12.24° ± 0.20°, 14.77° ± 0.20°, 16.55° ± 0.20°, 20.95° ± 0.20°, and 25.75° ± 0.20° by X-ray powder diffraction using Cu-Kα radiation.

Further preferably, the oxalate crystal form I has characteristic peaks at 2θ angles of 4.94° ± 0.20°, 5.26° ± 0.20°, 7.25° ± 0.20°, 12.24° ± 0.20°, 14.17° ± 0.20°, 14.77° ± 0.20°, 16.03° ± 0.20°, 16.55° ± 0.20°, 20.21° ± 0.20°, 20.95° ± 0.20°, 25.75° ± 0.20°, and 30.87° ± 0.20° by X-ray powder diffraction using Cu-Kα radiation.

Preferably, the oxalate crystal form I has characteristic peaks at 2θ angles by X-ray powder diffraction as shown in Table 14', with a tolerance range of ± 0.2°.

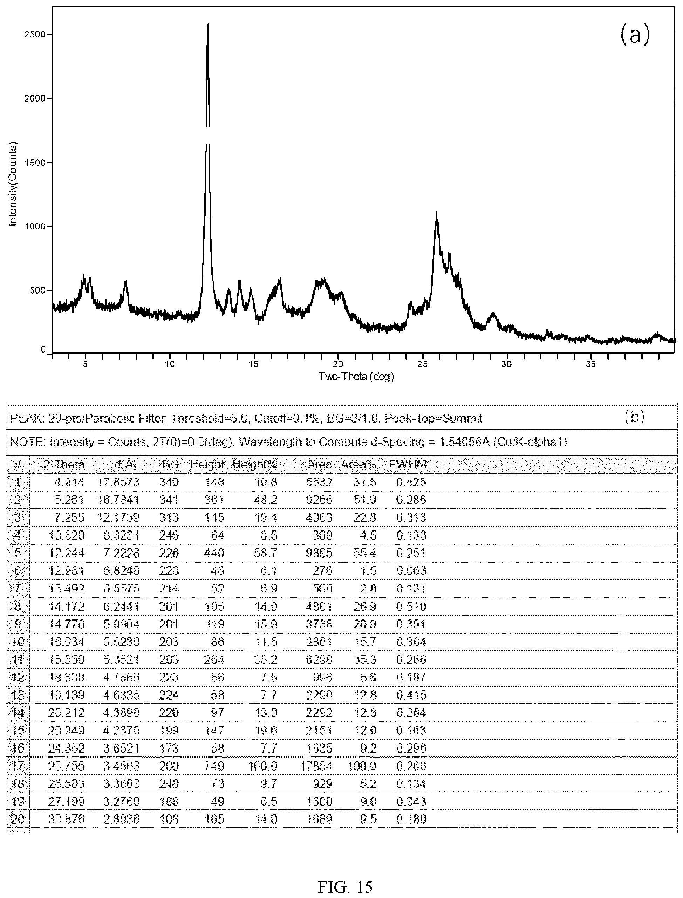

Preferably, the oxalate crystal form I has characteristic peaks at 2θ angles by X-ray powder diffraction as shown in FIG. 15(b), with a tolerance range of ± 0.20°.

According to a technical solution of the present disclosure, the oxalate crystal form I has an XRPD pattern substantially as shown in FIG. 15(a).

According to a technical solution of the present disclosure, in the oxalate crystal form I, compound A and the maleic acid are in a molar ratio of 1:1.

According to a technical solution of the present disclosure, the oxalate crystal form I is an anhydrate. According to a technical solution of the present disclosure, the oxalate crystal form I has a weight loss of about 0.5% at room temperature to about 150 °C.

In some embodiments, the oxalate crystal form I has a weight loss of 0.5% ± 0.4% at room temperature to 150 °C ± 3 °C.

According to a technical solution of the present disclosure, the oxalate crystal form I has a sharp endothermic peak with a peak temperature of about 206 °C.

In some embodiments, the oxalate crystal form I has a sharp endothermic peak with a peak temperature of 206 °C ± 5 °C, e.g., 206 °C ± 2 °C.

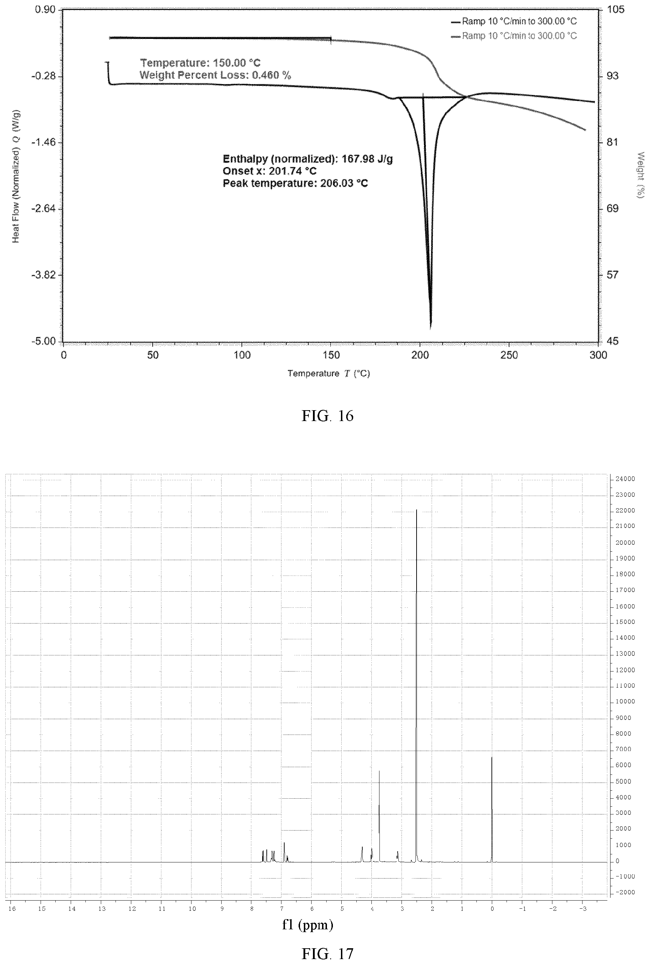

According to a technical solution of the present disclosure, the oxalate crystal form I has DSC and TGA patterns substantially as shown in FIG. 16.

According to a technical solution of the present disclosure, the oxalate crystal form I comprises no organic solvent.

According to a technical solution of the present disclosure, the fumarate salt of compound A comprises a salt formed from compound A and the fumaric acid according to a molar ratio of 1:1 or 2:1 (namely, the monofumarate salt of compound A or the hemifumarate salt of compound A).

According to a technical solution of the present disclosure, the fumarate salt may be in a crystal form selected from a fumarate crystal form I and a fumarate crystal form II.

According to a technical solution of the present disclosure, the fumarate crystal form I has characteristic peaks at 2θ angles of 3.90° ± 0.20°, 13.93° ± 0.20°, 16.86° ± 0.20°, and 26.37° ± 0.20° by X-ray powder diffraction using Cu-Kα radiation.

Preferably, the fumarate crystal form I has characteristic peaks at 2θ angles of 3.90° ± 0.20°, 10.45° ± 0.20°, 13.93° ± 0.20°, 16.86° ± 0.20°, 17.73° ± 0.20°, 21.39° ± 0.20°, 23.68° ± 0.20°, 26.37° ± 0.20°, 27.40° ± 0.20°, and 27.87° ± 0.20° by X-ray powder diffraction using Cu-Kα radiation.

Preferably, the fumarate crystal form I has characteristic peaks at 2θ angles by X-ray powder diffraction as shown in Table 19', with a tolerance range of ± 0.2°.

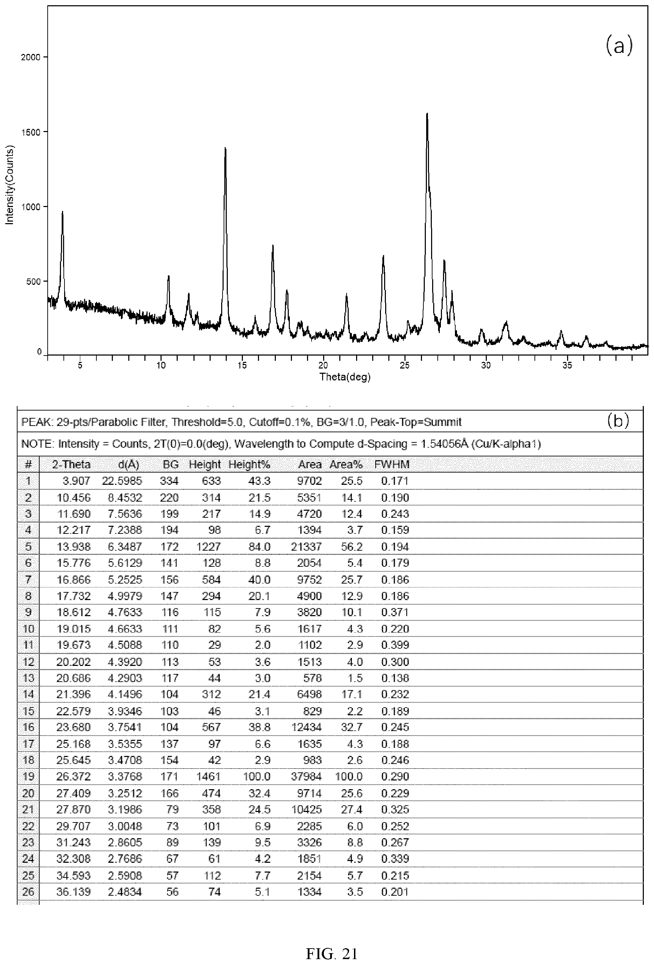

Preferably, the fumarate crystal form I has characteristic peaks at 2θ angles by X-ray powder diffraction as shown in FIG. 21(b), with a tolerance range of ± 0.20°.

According to a technical solution of the present disclosure, the fumarate crystal form I has an XRPD pattern substantially as shown in FIG. 21(a).

According to a technical solution of the present disclosure, in the fumarate crystal form I, compound A and the fumaric acid are in a molar ratio of 2:1.

According to a technical solution of the present disclosure, the fumarate crystal form I is an anhydrate. According to a technical solution of the present disclosure, the fumarate crystal form I has almost no weight loss at room temperature to about 150 °C.

In some embodiments, the fumarate crystal form I has almost no weight loss at room temperature to 150 °C ± 3 °C.

According to a technical solution of the present disclosure, the fumarate crystal form I has a sharp endothermic peak with a peak temperature of about 157 °C.

In some embodiments, the fumarate crystal form I has a sharp endothermic peak with a peak temperature of 157 °C ± 5 °C, e.g., 157 °C ± 2 °C.

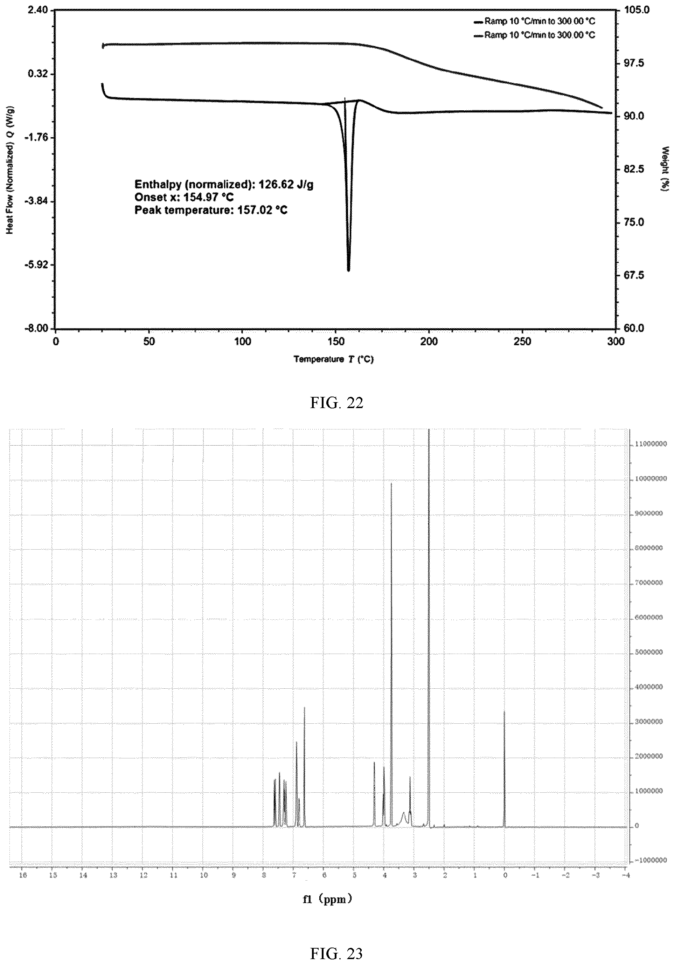

According to a technical solution of the present disclosure, the fumarate crystal form I has DSC and TGA patterns substantially as shown in FIG. 22.

According to a technical solution of the present disclosure, the fumarate crystal form I comprises no organic solvent.

According to a technical solution of the present disclosure, the fumarate salt of compound A is in a crystal form, which is named as a fumarate crystal form II. The fumarate crystal form II has characteristic peaks at 2θ angles of 22.06° ± 0.20° and 25.20° ± 0.20° by X-ray powder diffraction using Cu-Kα radiation.

Preferably, the fumarate crystal form II has characteristic peaks at 2θ angles of 22.06° ± 0.20°, 22.50° ± 0.20°, 25.20° ± 0.20°, and 27.54° ± 0.20° by X-ray powder diffraction using Cu-Kα radiation.

Further preferably, the fumarate crystal form II has characteristic peaks at 2θ angles of 11.44° ± 0.20°, 13.74° ± 0.20°, 22.06° ± 0.20°, 22.50° ± 0.20°, 24.60° ± 0.20°, 25.20° ± 0.20°, 27.54° ± 0.20°, and 28.78° ± 0.20° by X-ray powder diffraction using Cu-Kα radiation.

Preferably, the fumarate crystal form II has characteristic peaks at 2θ angles by X-ray powder diffraction as shown in Table 20', with a tolerance range of ± 0.2°.

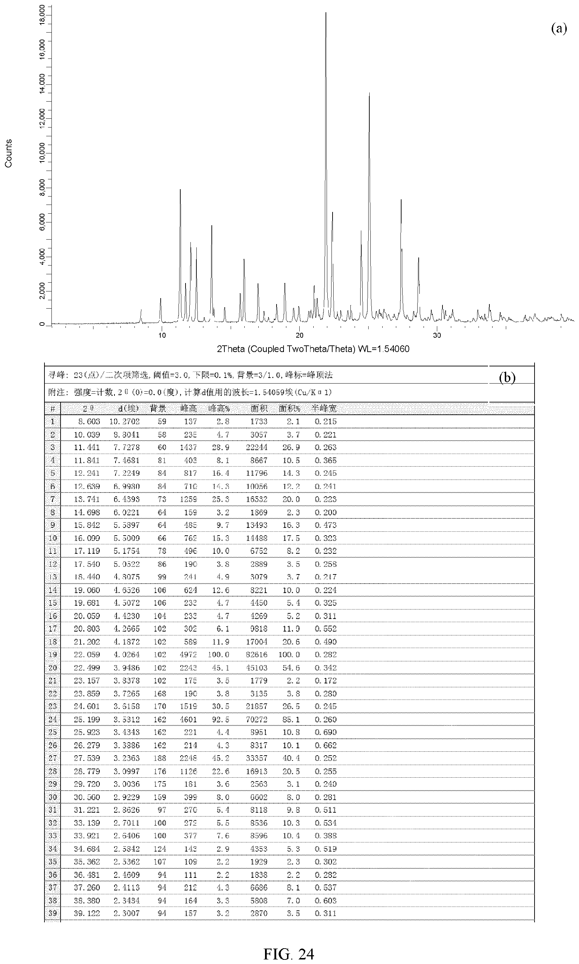

Preferably, the fumarate crystal form II has characteristic peaks at 2θ angles by X-ray powder diffraction as shown in FIG. 24(b), with a tolerance range of ± 0.20°.

According to a technical solution of the present disclosure, the fumarate crystal form II has an XRPD pattern substantially as shown in FIG. 24(a).

According to a technical solution of the present disclosure, in the fumarate crystal form II, compound A and the fumaric acid are in a molar ratio of 2:1.

According to a technical solution of the present disclosure, the fumarate crystal form II is an anhydrate.

According to a technical solution of the present disclosure, the fumarate crystal form II has almost no weight loss at room temperature to about 100 °C.

In some embodiments, the fumarate crystal form II has a weight loss of less than 0.1% at room temperature to 100 °C ± 3 °C.

According to a technical solution of the present disclosure, the fumarate crystal form II has a sharp endothermic peak with a peak temperature of about 181 °C.

In some embodiments, the fumarate crystal form II has a sharp endothermic peak with a peak temperature of 181 °C ± 5 °C, e.g., 181 °C ± 2 °C.

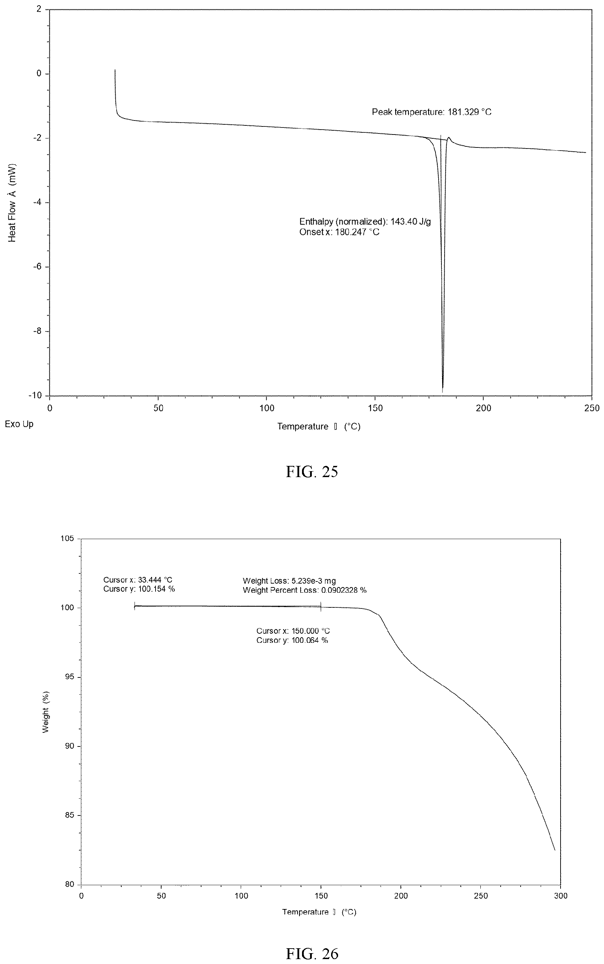

According to a technical solution of the present disclosure, the fumarate crystal form II has a TGA pattern substantially as shown in FIG. 25 and a DSC pattern substantially as shown in FIG. 26.

According to a technical solution of the present disclosure, the fumarate crystal form II contains an organic solvent. The organic solvent has a content of less than 0.5%, for example, less than 0.2%, illustratively 0.1%. The organic solvent is selected from one, two or more of the organic solvents shown below: ethanol (EtOH), methyl *tert*-butyl ether (MTBE), ethyl acetate (EA), acetonitrile (ACN), dichloromethane (DCM), acetone, and the like, illustratively acetone.

According to a technical solution of the present disclosure, the tartrate salt of compound A is in a crystal form, which is named as a tartrate crystal form I. The tartrate crystal form I has characteristic peaks at 2θ angles of 16.98° ± 0.20°, 17.85° ± 0.20°, 19.66° ± 0.20°, and 25.58° ± 0.20° by X-ray powder diffraction using Cu-Kα radiation.

Preferably, the tartrate crystal form I has characteristic peaks at 2θ angles of 13.38° ± 0.20°, 16.98° ± 0.20°, 17.85° ± 0.20°, 18.53° ± 0.20°, 19.66° ± 0.20°, 25.58° ± 0.20°, and 26.72° ± 0.20° by X-ray powder diffraction using Cu-Kα radiation.

Preferably, the tartrate crystal form I has characteristic peaks at 2θ angles of 13.38° ± 0.20°, 16.98° ± 0.20°, 17.27° ± 0.20°, 17.85° ± 0.20°, 18.53° ± 0.20°, 19.66° ± 0.20°, 20.46° ± 0.20°, 22.86° ± 0.20°, 25.58° ± 0.20°, 26.08° ± 0.20°, and 26.72° ± 0.20° by X-ray powder diffraction using Cu-Kα radiation.

Preferably, the tartrate crystal form I has characteristic peaks at 2θ angles by X-ray powder diffraction as shown in Table 22', with a tolerance range of ± 0.2°.

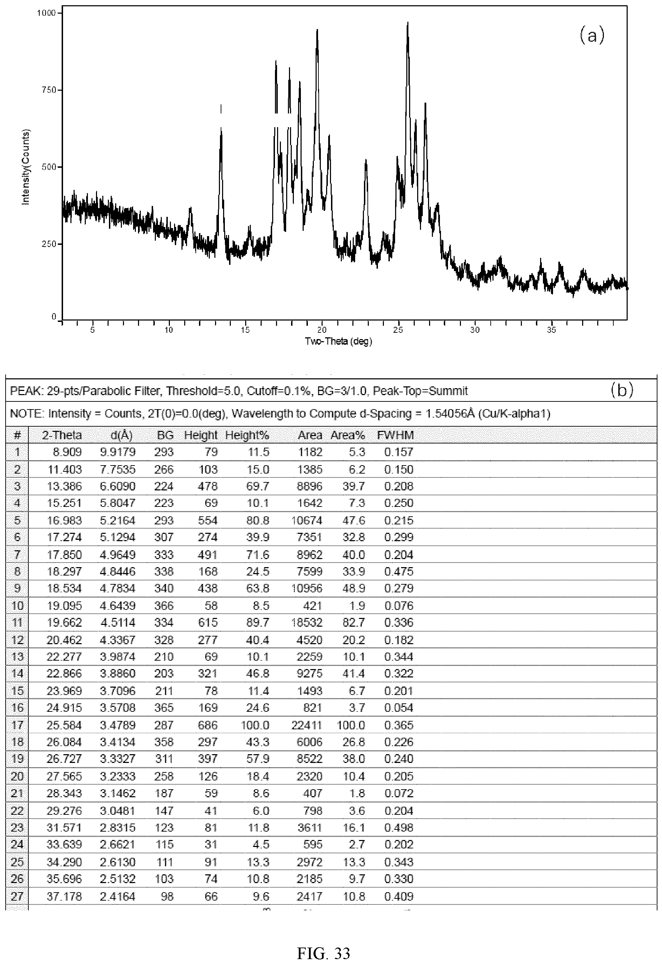

Preferably, the tartrate crystal form I has characteristic peaks at 2θ angles by X-ray powder diffraction as shown in FIG. 33(b), with a tolerance range of ± 0.20°.

According to a technical solution of the present disclosure, the tartrate crystal form I has an XRPD pattern substantially as shown in FIG. 33(a).

According to a technical solution of the present disclosure, in the tartrate crystal form I, compound A and the tartaric acid are in a molar ratio of 1:1.

According to a technical solution of the present disclosure, the tartrate crystal form I is an anhydrate. According to a technical solution of the present disclosure, the tartrate crystal form I has a weight loss of about 0.8% at room temperature to about 150 °C.

In some embodiments, the tartrate crystal form I has a weight loss of 0.8% ± 0.2% at room temperature to 150 °C ± 3 °C.

According to a technical solution of the present disclosure, the tartrate crystal form I has a sharp endothermic peak with a peak temperature of about 135 °C.

In some embodiments, the tartrate crystal form I has a sharp endothermic peak with a peak temperature of 135 °C ± 5 °C, e.g., 135 °C ± 2 °C.

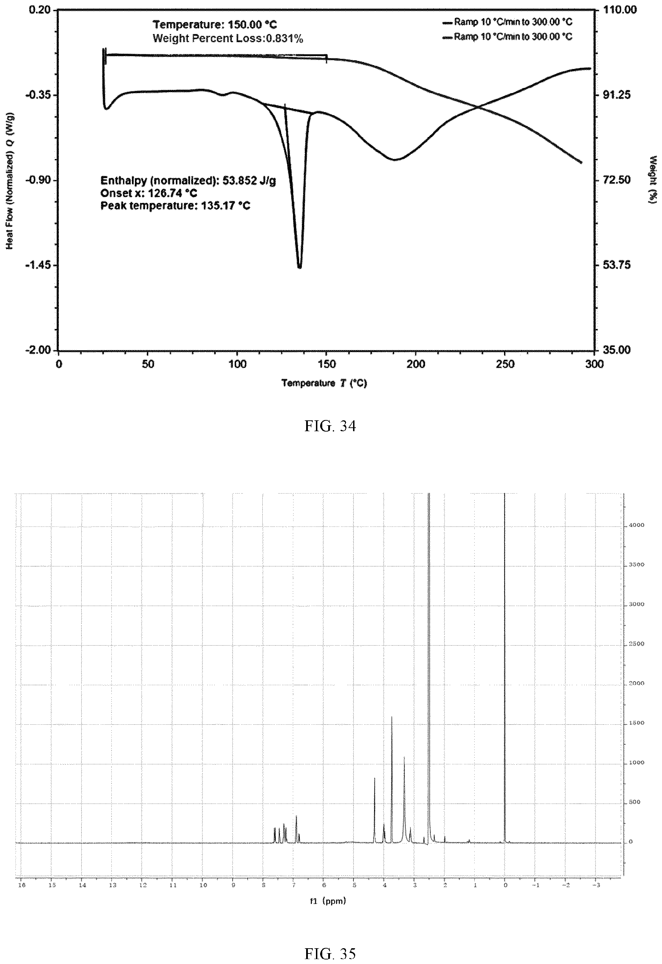

According to a technical solution of the present disclosure, the tartrate crystal form I has DSC and TGA patterns substantially as shown in FIG. 34.

According to a technical solution of the present disclosure, the tartrate crystal form I comprises no organic solvent. The organic solvent is selected from one, two or more of the organic solvents shown below: ethanol (EtOH), methyl *tert*-butyl ether (MTBE), ethyl acetate (EA), acetonitrile (ACN), dichloromethane (DCM), and the like, e.g., MTBE. Illustratively, the tartrate crystal form I comprises 0.8% MTBE.

The present disclosure also provides a preparation method for the salt of the ROCK inhibitor compound A described above, which comprises forming a salt of compound A with an acid. The acid is selected from hydrochloric acid, *p*-toluenesulfonic acid, benzenesulfonic acid, maleic acid, tartaric acid, oxalic acid, fumaric acid, sulfuric acid, methanesulfonic acid, phosphoric acid, succinic acid, and citric acid, and preferably is hydrochloric acid, *p*-toluenesulfonic acid, benzenesulfonic acid, maleic acid, tartaric acid, oxalic acid, or fumaric acid.

According to a technical solution of the present disclosure, the preparation method for the salt comprises the following steps: subjecting compound A to a salt-forming reaction with an acid in a solvent, stirring the mixture until a solid is precipitated, and drying the solid to obtain the salt;
if no solid is precipitated by stirring, adding an anti-solvent to the system, and drying a solid after the solid is precipitated to obtain the salt.

According to a technical solution of the present disclosure, the solvent may be selected from one, two or more of EA (ethyl acetate), 2-Me-THF (2-methyl-tetrahydrofuran), ACN (acetonitrile), DCM (dichloromethane), EtOH (ethanol), MeOH (methanol), IPA (isopropyl alcohol), THF (tetrahydrofuran), IPAc (isopropyl acetate), and MTBE, or a mixed solvent of any one, two or more of the solvents described above with MTBE (methyl *tert*-butyl ether). For example, the solvent is selected from EA, 2-Me-THF, ACN, DCM, MeOH, MTBE, IPA, EtOH, a mixed solvent of EA and MeOH, and a mixed solvent of MeOH, THF, and ACN.

According to a technical solution of the present disclosure, the anti-solvent may be selected from MTBE (methyl *tert*-butyl ether) and/or ACN (acetonitrile).

According to a technical solution of the present disclosure, compound A and the solvent are in a mass-to-volume ratio of 1 g:(5-50) mL, e.g., 1 g:(6-40) mL, illustratively 1 g:4.5 mL, 1 g:6.7 mL, 1 g: 7 mL, 1 g:10 mL, 1 g:12.5 mL, 1 g:15 mL, 1 g:17 mL, 1 g:20mL, 1 g:24 mL, 1 g:30 mL, or 1 g:36 mL. According to a technical solution of the present disclosure, compound A and the acid are in a molar ratio of 5:1-1:5, e.g., 1:(0.55-2.5), illustratively 1:0.55, 1:0.7, 1:0.75, 1:0.9, 1:1, 1:1.1, 1:1.2, or 1:2.2. According to a technical solution of the present disclosure, the anti-solvent and the solvent are in a volume ratio of (1-5):1, e.g., 2:1, 3:1, or 4:1.

According to a technical solution of the present disclosure, the stirring is performed at a temperature of 15-35 °C, preferably 20-25 °C.

According to a technical scheme of the present disclosure, the stirring is performed for 1-100 h, e.g., 3-80 h, illustratively 3 h, 5 h, 10 h, 15 h, 20 h, 30 h, 50 h, and 72 h.

According to some embodiments of the present disclosure, high-temperature stirring may also be performed before performing stirring at a temperature of 15-35 °C. For example, the high temperature is 60-70 °C; for example, the high-temperature stirring is performed for 0.5-2 h.

According to a technical scheme of the present disclosure, the drying is vacuum drying. The drying is performed for a period of time that can adjusted by those skilled in the art as desired. Preferably, the drying is performed for 3-15 h, and more preferably, the drying is performed for 3-10 h, e.g., 5-8 h. Preferably, the drying is performed at a temperature of 40-60 °C, e.g., 50 °C.

According to a preferred technical solution of the present disclosure, the preparation method comprises the following steps: dissolving or suspending compound A in the solvent, adding an acid to the system for a salt-forming reaction, stirring the mixture until a solid is precipitated, and drying the solid to obtain the salt;
if no solid is precipitated by stirring, adding an anti-solvent to the system, and drying a solid after the solid is precipitated to obtain the salt.

The acid is selected from hydrochloric acid, *p*-toluenesulfonic acid, benzenesulfonic acid, maleic acid, tartaric acid, oxalic acid, and fumaric acid.

Compound A and the solvent are in a mass-to-volume ratio of 1 g:(5-50) mL.

Compound A and the acid are in a molar ratio of 1 :(0.5-2.5).

The present disclosure also provides a pharmaceutical composition comprising the salt described above.

According to a technical solution of the present disclosure, the pharmaceutical composition may also comprise a pharmaceutically acceptable carrier. The pharmaceutically acceptable carrier may be selected from carriers known in the art, for example, including but not limited to one, two or more of an excipient, a lubricant, an adhesive, a disintegrant, an inorganic salt, a solvent, a dissolution aid, a suspending agent, an isotonic agent, a buffer, a preservative, an antioxidant, a colorant, a foaming agent, a flavoring agent, and the like.

According to a technical solution of the present disclosure, the pharmaceutical composition may also comprise a second active ingredient. For example, the second active ingredient is one, two or more of other ROCK inhibitors, tyrosine kinase inhibitors, tyrosinase inhibitors, inhibitors of profibrotic cytokines, serum amyloid P inhibitors, autotaxin-lysophosphatidic acid pathway inhibitors, GPR40 agonists, GPR84 antagonists, anti-acid drugs, and antibiotics.

The present disclosure also provides use of the salt or the pharmaceutical composition described above for manufacturing a preparation.

The present disclosure also provides a preparation comprising the salt.

Preferably, the preparation further comprises a pharmaceutically acceptable carrier.

Preferably, the preparation comprises the pharmaceutical composition.

According to a technical solution of the present disclosure, the preparation may be in the form of powders, tablets (such as coated tablets, and sustained-release or controlled-release tablets), lozenges, capsules (such as soft capsules or hard capsules), granules, pills, dispersible powders, suspensions, solutions, emulsions, elixirs, syrups, aerosols, creams, ointments, gels, injections, lyophilized powder injections, suppositories, or the like.

According to a technical solution of the present disclosure, the preparation may be applied in any one of the following modes: oral administration, buccal administration, sublingual administration, inhalation, topical application; intravenous, subcutaneous, acupoint or intramuscular injection by parenteral routes; and rectal administration.

According to a technical solution of the present disclosure, the preparation is a ROCK antagonist. Preferably, the ROCK antagonist is for use in the prevention and/or treatment of one or more diseases caused by high expression or excessive activation of ROCK.

For example, the disease is selected from cardiovascular and cerebrovascular diseases, neurological diseases, fibrosis diseases, ocular diseases, tumors, arterial thrombotic disorders, radiation damage, respiratory diseases, metabolic diseases, and autoimmune diseases. For example, the disease includes atherosclerosis, acute coronary syndrome, hypertension, cerebral vasospasm, cerebral ischemia, ischemic stroke, restenosis, heart disease, heart failure, cardiac hypertrophy, myocardial ischemia-reperfusion injury, diabetes, diabetic nephropathy, cancer, neuronal degeneration, nerve injury diseases, spinal cord injury, erectile dysfunction, platelet aggregation, leukocyte aggregation, glaucoma, ocular hypertension, asthma, osteoporosis, pulmonary fibrosis (such as idiopathic pulmonary fibrosis), hepatic fibrosis, renal fibrosis, COPD, kidney dialysis, glomerulosclerosis, fatty liver disease, fatty liver hepatitis, or neuronal degeneration inflammation.

The present disclosure also provides a method for preventing and/or treating diseases caused by high expression or excessive activation of ROCK, which comprises administering to a subject a therapeutically effective amount of the salt or the crystal form of the salt of compound A, the pharmaceutical composition, or the preparation.

### Description of Terms

The term "crystal form" refers to crystal forms that have identical chemical composition but different spatial arrangements of crystal-forming molecules and/or ions.

The term "amorphous form" refers to a solid form of molecules and/or ions that is not crystalline. An amorphous solid does not show a defined X-ray powder diffraction pattern with a clear maximum value.

Compound A is a free base, and the crystal form of the free base is the crystal form of compound A. The term "X-ray powder diffraction pattern substantially as shown in the figure" means that at least 50%, or at least 60%, or at least 70%, or at least 80%, or at least 90%, or at least 95%, or at least 99% of the main peaks shown in an X-ray powder diffraction pattern appear in the X-ray powder diffraction pattern. The main peak refers to a peak with a relative intensity greater than 10%, preferably greater than 20%, and more preferably greater than 30%, using the highest peak as a reference (the relative intensity of the highest peak is designated as 100%).

It can be understood by those skilled in the art that compounds may have multiple salt-forming sites, and thus, the salts of the compounds of the present disclosure include not only a salt formed at 1 salt-forming site of the compounds, but also salts formed at 2, 3 or all of the salt-forming sites of the compounds. For this purpose, the molar ratio of the compound to a radical ion (anion) of an acid required for salt formation may vary within a wide range and may be, for example, 5:1-1:5, e.g., 3:1, 2:1, 1:1, 1:1.5, 1:2, 1:2.5, 1:3, or the like.

The terms "object", "patient", and "subject" described herein have the same meaning and refer to a human or other warm-blooded mammals. The human described herein as a "subject" includes adults, infants and children, and the warm-blooded mammals include, but are not limited to, non-human primates such as chimpanzees, other apes or monkeys, other zoo animals, domestic animals or laboratory animals such as cats, pigs, dogs, cattle, sheep, mice, rats, and guinea pigs and the like. Preferably, the "subject" described herein is a human.

The term "effective amount" or "therapeutically effective amount" is an amount of a mixture, pharmaceutical composition, or preparation of either or both of the crystal forms I and II described herein in any ratio sufficient to effect the intended application (including but not limited to the disease treatment defined above), which may be determined according to the methods known to practicing physicians in the art. Determination of a therapeutically effective dose is well within the capability of the clinician or researcher in the art and may vary depending on the following factors: the intended application (*in vitro* or *in vivo*), or the subject and disease condition being treated, such as the weight and age of the subject, general health, severity of the disease or condition, mode of administration, and other factors affecting efficacy, such as drug allergy history. The specific administration dose will vary depending on the following factors: the selected particular compound or crystal form, the dosing regimen to be followed, whether to administer in combination with other compounds, the schedule of administration, the tissue to be administered, and the physical delivery system carried.

The term "room temperature" refers to a temperature of 15-30 °C, preferably 20-25 °C.

The term "hydrate" may include hemihydrate, monohydrate, dihydrate, trihydrate, and tetrahydrate. The crystal forms of compound A of the present disclosure include non-solvate (anhydrate) and solvate (solvate-containing) crystal forms of compound A.

The term "about" used herein refers to haply, roughly, approximately, or "in the region of ...". The term "about", when used in conjunction with a numerical range, modifies this range by extending the boundaries above and below the numerical values set forth. Generally, the term "about" is used herein to modify such values within reasonable limits for such parameters as would be understood by those skilled in the art. Specifically, when "about" is used in conjunction with a range of temperature, it means that the temperature fluctuates within, for example, a range of ± 5°C, ± 4°C, ± 3°C, ± 2°C, or ± 1°C. When "about" is used in conjunction with a range representing weight loss, it optionally means that the weight fluctuates within, for example, a range of ± 5%, ± 4%, ± 3%, ± 2%, ± 1%, or ± 0.4%. Herein, "almost no weight loss" refers to a percentage of weight loss of less than 0.2%, preferably less than 0.1%.

The "%" content of the organic solvent refers to the mass percentage content of organic solvent.

### BRIEF DESCRIPTION OF THE DRAWINGS

FIG. 1 shows an XRPD pattern (a) and pattern analysis (b) of a hydrochloride crystal form I.
FIG. 2 shows DSC and TGA patterns of a hydrochloride crystal form I.
FIG. 3 shows a ¹H-NMR spectrum of a hydrochloride crystal form I.
FIG. 4 shows an XRPD pattern (a) and pattern analysis (b) of a *p*-toluenesulfonate crystal form I.
FIG. 5 shows DSC and TGA patterns of a *p*-toluenesulfonate crystal form I.
FIG. 6 shows a ¹H-NMR spectrum of a *p*-toluenesulfonate crystal form I.
FIG. 7 shows an XRPD pattern (a) and pattern analysis (b) of a benzenesulfonate crystal form I.
FIG. 8 shows DSC and TGA patterns of a benzenesulfonate crystal form I.
FIG. 9 shows a ¹H-NMR spectrum of a benzenesulfonate crystal form I.
FIG. 10 shows an XRPD pattern (a) and pattern analysis (b) of a maleate crystal form I.
FIG. 11 shows an XRPD pattern (a) and pattern analysis (b) of a maleate crystal form II.
FIG. 12 shows DSC and TGA patterns of a maleate crystal form I.
FIG. 13 shows DSC and TGA patterns of a maleate crystal form II.
FIG. 14-1 shows a ¹H-NMR spectrum of a maleate crystal form I.
FIG. 14-2 shows a ¹H-NMR spectrum of a maleate crystal form II.
FIG. 15 shows an XRPD pattern (a) and pattern analysis (b) of an oxalate crystal form I.
FIG. 16 shows DSC and TGA patterns of an oxalate crystal form I.
FIG. 17 shows a ¹H-NMR spectrum of an oxalate crystal form I.
FIG. 18 shows an XRPD pattern of an oxalate crystal form I obtained by scale-up preparation.
FIG. 19 shows an overlay of DSC and TGA patterns of an oxalate crystal form I obtained by scale-up preparation.
FIG. 20 shows a ¹H-NMR spectrum of an oxalate crystal form I obtained by scale-up preparation.
FIG. 21 shows an XRPD pattern (a) and pattern analysis (b) of a fumarate crystal form I.
FIG. 22 shows DSC and TGA patterns of a fumarate crystal form I.
FIG. 23 shows a ¹H-NMR spectrum of a fumarate crystal form I.
FIG. 24 shows an XRPD pattern (a) and pattern analysis (b) of a fumarate crystal form II.
FIG. 25 shows a TGA pattern of a fumarate crystal form II.
FIG. 26 shows a DSC pattern of a fumarate crystal form II.
FIG. 27 shows a ¹H-NMR spectrum of a fumarate crystal form II.

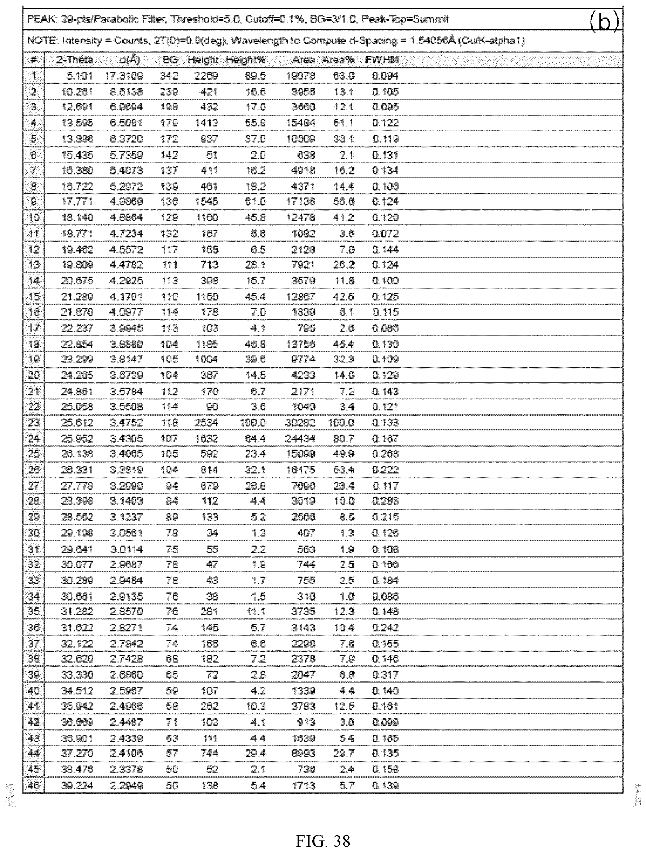

FIG. 28 shows an HPLC purity test of a fumarate crystal form II obtained from group 1 in Example 8.
FIG. 29 shows an HPLC purity test of a fumarate crystal form II obtained from group 4 in Example 8.
FIG. 30 shows an XRPD pattern of a fumarate crystal form I obtained by scale-up preparation in Example 9.
FIG. 31 shows an overlay of DSC and TGA patterns of a fumarate crystal form I obtained by scale-up preparation.
FIG. 32 shows a ¹H-NMR spectrum of a fumarate crystal form I obtained by scale-up preparation.
FIG. 33 shows an XRPD pattern (a) and pattern analysis (b) of a tartrate salt.
FIG. 34 shows DSC and TGA patterns of a tartrate crystal form I.
FIG. 35 shows a ¹H-NMR spectrum of a tartrate crystal form I.
FIG. 36 shows an XRPD pattern (a) and pattern analysis (b) of a free base crystal form I.
FIG. 37 shows DSC and TGA patterns of a free base crystal form I.
FIG. 37-2 shows a ¹H-NMR spectrum of a free base crystal form I.
FIG. 38 shows an XRPD pattern (a) and pattern analysis (b) of a free base crystal form II.
FIG. 39 shows DSC and TGA patterns of a free base crystal form II.
FIG. 39-1 shows a ¹H-NMR spectrum of a free base crystal form II.
FIG. 40 shows a DVS pattern of a free base crystal form II.
FIG. 41 shows a DVS pattern of an oxalate crystal form I.
FIG. 42 shows a DVS pattern of a fumarate crystal form I.
FIG. 43 shows a DVS pattern of a fumarate crystal form II.
FIG. 44 shows an XRPD pattern of a fumarate crystal form II before and after DVS test.

### DETAILED DESCRIPTION

The embodiments of the present disclosure will be further illustrated in detail with reference to the following specific examples. It should be understood that the following examples are merely exemplary illustrations and explanations of the present disclosure, and should not be construed as limiting the protection scope of the present disclosure. All techniques implemented based on the content of the present disclosure described above are included within the protection scope of the present disclosure.

Unless otherwise stated, the starting materials and reagents used in the following examples are all commercially available products or can be prepared using known methods.

Characterization and tests involved in the following examples:

### X-Ray Powder Diffractometer (XRPD)

The solid product obtained in the experiment was subjected to solid morphology analysis using an X-ray powder diffractometer PANalytical Empyrean equipped with a PIXcel1D detector. The target of the X-ray tube of the instrument used was a copper target (K-Alpha (λ = 1.5418 Å)). The light tube voltage and current were 45 kV and 40 mA, respectively. The scanning range for the sample was 3° 2θ to 40° 2θ, with a step size of 0.013° 2θ. The sample tray speed and testing speed were 60 rpm and 0.164° 2θ/s, respectively.

### Differential Scanning Calorimetry (DSC)

The sample was subjected to thermal analysis using Discovery DSC 250 (TA Instruments, US). An appropriate amount of the sample was weighed and added to a DSC sample tray, which was then pricked. The sample was heated to the final temperature at a rate of 10 °C/min after equilibration at 25 °C.

### Thermogravimetric Analysis (TGA)

The sample was subjected to thermogravimetric analysis using TGA 55 (TA Instruments, US). The sample was placed in a tared closed aluminum sample tray, and after the sample mass was automatically measured in a TGA furnace, the sample was heated from room temperature to the final temperature at a rate of 10 °C/min.

### Hydrogen Nuclear Magnetic Resonance Spectroscopy (¹H-NMR)

The hydrogen spectrum information of the sample was confirmed by ¹H-NMR. The instrument used for ¹H-NMR analysis was Bruker AVANCE III HD 300/400 equipped with a Sample Xpress 60 autosampler system.

### Dynamic Vapor Sorption (DVS)

The sample was subjected to water adsorption/desorption tests using a Vsorp (ProUmid GmbH & Co. KG, Germany) water adsorption analyzer. The sample was placed in a tared sample tray, and the change in the sample mass with humidity (0-90% RH) at 25 °C was recorded. The specific DVS test parameters are shown in Table 1 below.

**Table 1. Hygroscopicity method in the DVS test**

| | |
|---|---|
| Equilibrium condition | 0.01% /45min |
| Time of cyclic weighing | 10 min |
| Minimum time interval | 50 min |
| Maximum time interval | 2.0 h |
| Equilibrium condition | 40 °C @ 0% RH (relative humidity) for 6 h |
| Sampling temperature | 25 °C |
| Adsorption humidity | 0, 10, 20, 30, 40, 50, 60, 70, 80, 90 %RH |
| Desorption humidity | 80, 70, 60, 50, 40, 30, 20, 10, 0 %RH |

### High Performance Liquid Chromatography (HPLC)

The instrument used for HPLC analysis was Agilent HPLC 1260 series. The HPLC method used for a solubility test is shown in Table 1-1. The HPLC method used for a stability test is shown in Tables 2 and 3.

**Table 1-1. HPLC method for the solubility test**

| | | |
|---|---|---|
| Instrument | Aglient NB-MS-HPLC-3 | |
| Chromatographic column | YMC-pack Pro C18 150 mm*4.6 mm S-3µm, 8 nm | |
| Mobile phase | A: water, B: ACN (acetonitrile) | |
| Gradient (TB%) | Time [min] | B [%] |
| | 0.00 | 10.0 |
| | 9.00 | 90.0 |
| | 10.00 | 90.0 |
| | 10.10 | 10.0 |
| Column temperature | 30 °C | |
| Detector | DAD, 265 nm | |
| Flow rate | 1.0 mL/min | |
| Injection volume | 5 µL | |
| Elution time | 10.10 min | |
| Diluent | ACN/water (1/1, v/v) | |

**Table 2. HPLC method for a 7-day stability test**

| | | |
|---|---|---|
| Instrument | Aglient NB-MS-HPLC-2 | |
| Chromatographic column | YMC-pack Pro C18 150 mm*4.6 mm S-5µm, 12 nm | |
| Mobile phase | A: 0.05% aqueous TFA (trifluoroacetic acid) solution; | |
| | B: 0.05% TFA in ACN | |
| Gradient (TB%) | Time [min] | B [%] |
| | 0.00 | 10.0 |
| | 2.00 | 30.0 |
| | 11.00 | 45.0 |
| | 13.00 | 90.0 |
| Column temperature | 35 °C | |
| Detector | DAD, 265 nm | |
| Flow rate | 1.0 mL/min | |
| Injection volume | 2 µL | |
| Elution time | 13 min | |
| Diluent | ACN/water (1/1, v/v) | |

**Table 3. HPLC method for a 14-day stability test**

| | | |
|---|---|---|
| Instrument | Aglient NB-MS-HPLC-2 | |
| Chromatographic column | YMC-pack Pro C18 150 mm*4.6 mm S-5µm, 12 nm | |
| Mobile phase | A: 0.05% aqueous TFA solution; | |
| | B: 0.05% TFA in ACN | |
| Gradient (TB%) | Time [min] | B [%] |
| | 0.00 | 10.0 |
| | 2.00 | 30.0 |
| | 11.00 | 45.0 |
| | 13.00 | 90.0 |
| | 23.00 | 90.0 |
| Column temperature | 35 °C | |
| Detector | DAD, 265 nm | |
| Flow rate | 1.0 mL/min | |
| Injection volume | 2 µL | |
| Elution time | 23 min | |
| Diluent | ACN/water (1/1, v/v) | |

### Ion Chromatography (IC)

The instrument used for IC analysis was Thermo ICS-6000. The method used for the ion chromatography test is shown in Table 4.

**Table 4. Parameters for the ion chromatography test method (Cl⁻ and C₂O₄²⁻)**

| | |
|---|---|
| Instrument | Thermo ICS-6000 |
| Work station | Chomeleon Workstation |
| Eluent generator | EGC 500 KOH |
| Suppressor | Dionx ASRS 300 4 mm |
| Guard column | Dionex IonPacTMAG11-HC (4*50 mm) |
| Chromatographic column | Dionex IonPacTMAG11-HC (4*250 mm) |
| Temperature of conductance cell | 35.0 °C |
| Concentration of eluent | 30 mm |
| Working mode of suppressor | External mode |
| Current of suppressor | 75 mA |
| Column temperature | 30.0 °C |
| Flow rate | 1.0 mL/min |
| Elution gradient | Isocratic elution |
| Run time | 10 min |
| Injection volume | 25 µL |
| Flow rate of external water circulation | 1.5 mL/min |

### Polarizing Microscope (PLM) Analysis

The instrument used for PLM was Polarizing Microscope ECLIPSE LV100POL (Nikon, JPN).

### Laser Particle Size Analyzer (PSD)

The instrument used for the PSD analysis was Mastersizer3000. The method used for the test is shown in Table 4'.

**Table 4'. Parameters for laser particle size test method**

| | | |
|---|---|---|
| Instrument | Mastersizer3000 | |
| Test range | 0.01-3500µm | |
| Sample information | Particle type | Non-spherical |
| | Material name | Organic compound |
| | Refractive index | 1.59 |
| | Absorption index | 0.1 |
| Duration | Red background time | 10s |
| | Red measurement time | 10s |
| Test program | Number of measurements | 3 |
| | Delay between measurement: | 0s |
| Obscuration | Particles < 10 µm | 5% -10% |
| | Particles ≥ 10 µm | 5% -20% |
| Sample dispersion method | External sonication | 30s |
| | Internal sonication | N/A |
| | Dispersant | Water |
| | Dispersant refractive index | 1.33 |
| | Pre-dispersant | 0.1% Tween 80 aqueous solution |
| | Stirrer speed | 2000 rpm |
| | Tank fill | Manual |
| | Degas after fill | Yes |
| Cleaning type | None | |
| Analysis mode | General purpose | |
| Result type | Volume distribution | |

### Preparation Example: Preparation of compound A

Preparation of compound 5-(3-amino-1*H*-pyrazol-4-yl)-6-fluoro-*N*-(3-methoxybenzyl)indoline-1-carboxamide (compound A)

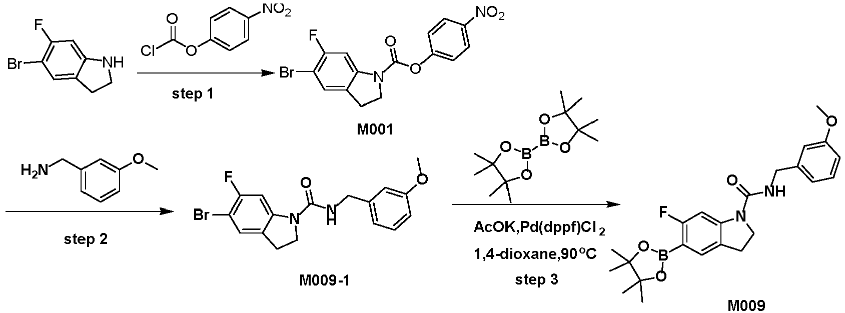

### (1) Preparation of compound 4-nitrophenyl 5-bromo-6-fluoroindoline-1-carboxylate (M001)

4-Nitrophenyl chloroformate (CAS number: 7693-46-1, 6.21 g) was dissolved in dichloromethane (40 mL). The resulting solution was cooled to 0 °C, and a solution of 5-bromo-6-fluoroindoline (6.00 g) and pyridine (8.86 g) in methylene chloride (50 mL) was added dropwise. The mixture was heated to room temperature and stirred overnight (15 h). Dichloromethane (100 mL) was added to the reaction liquid for dilution. Then, the mixture was washed with saturated brine (50 mL × 2), dried over anhydrous sodium sulfate, and filtered, and the filtrate was concentrated. The resulting crude product was separated by silica gel column chromatography (petroleum ether (PE):dichloromethane = 3:1, volume ratio) to give a gray solid as compound M001 (7.20 g, yield: 68%). LC-MS [M+H]⁺ = 380.9.

### (2) Preparation of compound 5-bromo-6-fluoro-N-(3-methoxybenzyl)indoline-1-carboxamide (M009-1)

Compound M001(1600 mg) and 3-methoxybenzylamine (1150 mg) were added to THF (tetrahydrofuran, 20 mL), and then *N,N*-diisopropylethylamine (2714 mg) was added to the resulting solution under stirring at room temperature. The resulting reaction liquid was stirred at 75 °C for 15 h in an oil bath. After the reaction was completed, the reaction liquid was concentrated under reduced pressure. The resulting crude product was purified by silica gel column chromatography (petroleum ether:ethyl acetate = 3:1, volume ratio) to give a yellow solid as compound M009-1 (1500 mg, yield: 94.2%), LC-MS [M+H]⁺ = 381.1.

### (3) Preparation of compound 6-fluoro-N-(3-methoxybenzyl)-5-(4,4,5,5-tetramethyl-1,3,2-dioxaborolan-2-yl)indoline-1-carboxamide (M009)

Compound M009-1 (1500 mg), bis(pinacolato)diboron (CAS: 73183-34-3, 2010 mg), potassium acetate (AcOK, 1940 mg), and [1,1'-bis(diphenylphosphino)ferrocene]dichloropalladium(II) (Pd(dppf)Cl₂, 579 mg) were added to 1,4-dioxane (20 mL) under nitrogen atmosphere. The resulting reaction liquid was stirred at 90 °C for 5 h in an oil bath. After the reaction was completed, the reaction liquid was concentrated. The resulting crude product was purified by silica gel column chromatography (dichloromethane:methanol = 50:1, volume ratio) to give a yellow oil as compound M009 (800 mg, yield: 47.4%), LC-MS [M+H]⁺ = 427.1.

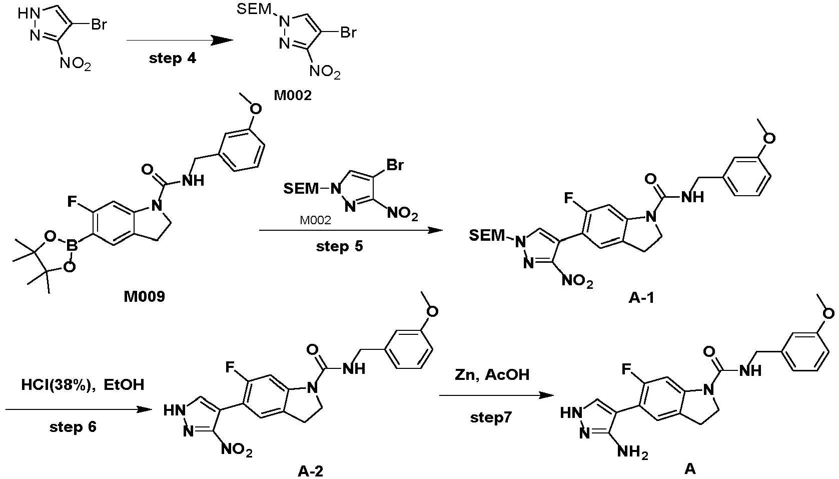

### (4) Synthesis of 4-bromo-3-nitro-1-((2-(trimethylsilyl)ethoxy)methyl)-1H-pyrazole (M002)

4-Bromo-3-nitro-1*H*-pyrazole (CAS number: 89717-64-6, 40 g) was weighed and dissolved in THF (400 mL). The resulting solution was cooled and maintained at 0-5 °C, and NaH (12.5 g) was added in 2-4 batches. The mixture was maintained at 0-5 °C for 0.5 h, and 2-(trimethylsilyl)ethoxymethyl chloride (SEM-Cl) (41.6 g) was added dropwise. Then, the reaction liquid was heated to room temperature and reacted for 2 h at this temperature. Water (600 mL) was added to the reaction liquid. The mixture was extracted once with EA (500 mL) and twice with EA (300 mL). The organic phase was taken, washed once with an ammonium chloride solution (300 mL) and saturated brine (300 mL) sequentially, dried over anhydrous sodium sulfate, and concentrated to dryness to give a crude product (70.2 g). *n*-Heptane (50 mL) was added to the crude product. The resulting mixture was slurried for 3 h at room temperature and rinsed with PE (50 mL) to give a white solid as compound M002 (51.2 g, yield:76%, HPLC purity: 96.8%), LC-MS [M+H]⁺ = 322.0.

### (5) Preparation of compound 6-fluoro-N-(3-methoxybenzyl)-5-(3-nitro-1-(((2-(trimethylsilyl)ethoxy)methyl)-1H-pyrazol-4-yl)indoline-1-carboxamide (A-1)

Compound M009 (800 mg), 4-bromo-3-nitro-1-(((2-(trimethylsilyl)ethoxy)methyl)-1*H*-pyrazole (544 mg), anhydrous potassium carbonate (1040 mg), and [1,1'-bis(diphenylphosphino)ferrocene]dichloropalladium(II) (137 mg) were added to 1,4-dioxane/water (20:1, 10 mL) under nitrogen atmosphere. The resulting reaction liquid was stirred at 80 °C for 2 h in an oil bath. After the reaction was completed, water (50 mL) was added to the reaction liquid for dilution. The resulting mixture was extracted with ethyl acetate (30 mL ×3). The organic phases were combined. The resulting organic phase was washed with saturated brine (20 mL), dried over anhydrous sodium sulfate, and filtered, and the filtrate was concentrated. The residue was purified by silica gel column chromatography (petroleum ether:ethyl acetate = 20:1, volume ratio) to give a yellow oil as compound A-1 (650 mg, yield 63.9%), LC-MS [M+H]⁺ = 542.1.

### (6) Preparation of compound 6-fluoro-N-(3-methoxybenzyl)-5-(3-nitro-1H-pyrazol-4-yl)indoline-1-carboxamide (A-2)

Compound A-1 (650 mg) was dissolved in ethanol (10 mL), and concentrated hydrochloric acid (1 mL, 38%) was added to the resulting solution. The resulting reaction liquid was stirred under reflux at 80 °C for 5 h in an oil bath. After the reaction was completed, compound A-2 was obtained, which was directly used in the next step without treatment. LC-MS [M+H]⁺ = 412.1.

### (7) Preparation of compound 5-(3-amino-1H-pyrazol-4-yl)-6-fluoro-N-(3-methoxybenzyl)indoline-1-carboxamide (compound A)

Activated zinc powder (Zn, 798 mg) was added to the reaction liquid obtained in step (6) in an ice-water bath, and then acetic acid (AcOH, 3 mL) was added. The resulting reaction liquid was cooled to room temperature, stirred for 2 h, and concentrated under reduced pressure. Then, saturated sodium bicarbonate (10 mL) was added, and the resulting mixture was extracted with ethyl acetate (5 mL × 3). The combined organic phase was dried over anhydrous sodium sulfate and filtered, and the filtrate was concentrated. The resulting crude product was purified by silica gel column chromatography (dichloromethane:methanol 20:1) to give compound A as a white solid (98 mg, two-step yield: 21.4%), LC-MS [M+H]⁺ = 382.2;
¹H NMR (400 MHz, DMSO-*d₆*) δ 11.69 (s, 1H), 7.61 (d, J = 12.9 Hz, 1H), 7.46 (s, 1H), 7.31 (dd, J = 11.5, 6.1 Hz, 2H), 7.24 (t, J = 8.0 Hz, 1H), 6.93 - 6.87 (m, 2H), 6.80 (dd, J = 7.3, 1.9 Hz, 1H), 4.59 (s, 2H), 4.31 (d, J = 5.8 Hz, 2H), 3.99 (t, J = 8.7 Hz, 2H), 3.74 (s, 3H), 3.12 (t, J = 8.5 Hz, 2H).

### Example 1: Preparation of hydrochloride salt

An appropriate amount (20-30 mg) of compound A was weighed and added to a sample flask at room temperature. Then, 0.2 mL of each of the different solvents shown in Table 5 was added, and 1 M hydrochloric acid was added for a salt-forming reaction. The resulting mixture was stirred at room temperature for 3-6 h. If no solid is precipitated, an anti-solvent (MTBE) is added to promote precipitation. If there is a solid, the sample is collected by filtration, dried under vacuum at 50 °C for about 3 h, and characterized by XRPD, TGA, DSC, and ¹H-NMR. Specific starting material information and results are listed in Table 5. The XRPD pattern and analysis of a hydrochloride crystal form I prepared from group 1 are shown in FIG. 1 and Table 5'.

**Table 5. Preparation of hydrochloride salt**

| **Group** | **Solvent** | **Volume of solvent/mass of compound A (mL/g)** | **Acid (µL)** | **Anti-solvent (v/vₐₙₜᵢ)** | **Result** |
|---|---|---|---|---|---|
| 1 | EA | 6.7 | 86 | N/A | Hydrochloride crystal form I |
| 2 | 2-Me-THF | 6.7 | 86 | | Hydrochloride crystal form I |
| 3 | ACN | 10 | 52 | | Hydrochloride crystal form I |
| 4 | DCM | 10 | 52 | | Hydrochloride crystal form I |
| 5 | EA | 6.7 | 172 | | Hydrochloride crystal form I |

**Table 5'. XRPD analysis of hydrochloride crystal form I**

| **20/°** | **Relative intensity I/%** | **20/°** | **Relative intensity I/%** |
|---|---|---|---|
| 5.927 | 100.0 | 21.021 | 5.2 |
| 11.957 | 22.7 | 24.074 | 84.9 |
| 12.556 | 8.3 | 25.533 | 7.4 |
| 14.922 | 49.3 | 26.610 | 23.1 |
| 17.981 | 26.7 | 27.186 | 21.5 |
| 18.964 | 8.0 | 31.662 | 8.7 |

In this example, one crystal form of the hydrochloride salt was obtained, which was named as a hydrochloride crystal form I. The characterization results of TGA, DSC, ¹H-NMR, and IC are summarized in Table 6 and FIGs. 2-3.

The hydrochloride crystal form I had a weight loss of about 5.4% before 110 °C. ¹H-NMR analysis showed that there were no organic solvent residues in the sample, so TGA weight loss was attributed to the removal of water (about 1 equivalent of H₂O). There were multiple thermodynamic events in the DSC pattern, wherein a broad endothermic peak with a peak temperature of about 97 °C was attributed to the removal of water. IC analysis showed that this sample contained about 1 equivalent of chloride ions, so the salt-forming ratio was 1/1. The hydrochloride crystal form I was determined to be a hydrate.

**Table 6. Solid state characterization results for hydrochloride crystal form I**

| **Crystal form solvation** | **DSC, endo Onset/Peak (°C), ΔH (J/g)** | **TGA Wt. loss %/ @T (°C)** | **¹H-NMR** | **IC acid/base ratio** |
|---|---|---|---|---|
| Hydrochloride crystal form I hydrate | 83/97, 144 | 5.4/RT-110 | No organic solvent residues | 1:1 |

**Table 7. IC data for hydrochloride crystal form I**

| **Sample** | **Mass (mg)** | **Theoretical concentration (µg/mL)** | **IC peak area (µS*min)** | **Note** |
|---|---|---|---|---|
| Hydrochloride crystal form I | 2.84 | 9.6 | 2.2061 | About 1 equivalent of Cl⁻ |

### Example 2: Preparation of p-toluenesulfonate salt

An appropriate amount (20-30 mg) of compound A was weighed and added to a sample flask at room temperature, and then dissolved or suspended in 0.2 mL of the solvent selected from Table 8. A *p-*toluenesulfonic acid solid or a 1 M solution of *p*-toluenesulfonic acid in methanol was added for a salt-forming reaction. The solution or suspension was stirred at room temperature for 5-15 h. If no solid is precipitated, an anti-solvent is added to promote precipitation. If there is a solid, the sample is collected by filtration and washing, dried under vacuum at 50 °C for about 3 h, and characterized by XRPD, TGA, DSC, and ¹H-NMR. Specific information and results are listed in Table 8. The XRPD pattern and analysis of a p-toluenesulfonate crystal form I prepared from group 1 are shown in FIG. 4 and Table 8'.

**Table 8. Preparation of p-toluenesulfonate salt**

| **Group** | **Solvent** | **Volume of solvent/mass of compound A (mL/g)** | **Acid** | **Result** |
|---|---|---|---|---|
| 1 | EtOH | 6.7 | 15 mg | *p*-Toluenesulfonate crystal form I |
| 2 | EA | 6.7 | | *p*-Toluenesulfonate crystal form I |
| 3 | 2-Me-THF | 6.7 | | Oil |
| 4 | ACN | 10 | | *p*-Toluenesulfonate crystal form I |
| 5 | DCM | 10 | 52 µL | *p*-Toluenesulfonate crystal form I |

**Table 8'. XRPD analysis of p-toluenesulfonate crystal form I**

| **2θ/°** | **Relative intensity I/%** | **2θ/°** | **Relative intensity I/%** |
|---|---|---|---|
| 5.495 | 33.0 | 19.912 | 50.5 |
| 7.555 | 93.7 | 20.675 | 30.3 |
| 8.608 | 100.0 | 22.344 | 13.6 |
| 9.145 | 31.9 | 22.709 | 7.3 |
| 10.052 | 35.9 | 23.380 | 60.6 |
| 11.193 | 12.6 | 24.022 | 49.0 |
| 12.035 | 25.8 | 24.600 | 40.6 |
| 12.363 | 10.9 | 25.205 | 21.9 |
| 12.706 | 10.2 | 25.743 | 6.4 |
| 13.755 | 23.5 | 25.989 | 8.9 |
| 14.397 | 32.5 | 26.269 | 7.9 |
| 14.752 | 64.7 | 26.594 | 12.4 |
| 15.999 | 65.3 | 26.938 | 7.5 |
| 16.734 | 28.2 | 28.013 | 13.2 |
| 17.167 | 20.2 | 28.251 | 19.9 |
| 17.431 | 15.0 | 29.696 | 4.5 |
| 17.876 | 17.2 | 30.301 | 4.3 |
| 18.101 | 12.4 | 31.056 | 5.2 |
| 18.638 | 28.8 | 32.002 | 4.8 |
| 19.191 | 13.9 | 32.702 | 3.6 |
| 19.637 | 46.0 | 34.279 | 7.7 |

In this example, one crystal form of the *p*-toluenesulfonate salt was obtained, which was named as a *p*-toluenesulfonate crystal form I. The characterization results of TGA, DSC, and ¹H-NMR are summarized in Table 9 and FIGs. 5-6.

The *p*-toluenesulfonate crystal form I had a weight loss of about 2.5% before 105 °C. ¹H-NMR analysis showed that the sample contained 0.5% EtOH and 0.1% MTBE organic solvent, so the TGA weight loss was attributed to the removal of water and organic solvent. There were two thermodynamic events in the DSC pattern, wherein a broad endothermic peak with a peak temperature of about 89 °C was attributed to the removal of water, and the second endothermic peak with a peak temperature of about 127 °C was determined to be caused by melting. Thus, the *p*-toluenesulfonate crystal form I was determined to be a hydrate.

**Table 9. Solid state characterization results for p-toluenesulfonate crystal form I**

| **Crystal form solvation** | **DSC, endo Onset/Peak(°C), ΔH (J/g)** | **TGA Wt. loss%/ @T (°C)** | **¹H-NMR** |
|---|---|---|---|
| *p-*Toluenesulfonate crystal form I hydrate | 40/89, 42 | 2.5/RT - 105 | 0.5% EtOH and 0.1% MTBE; |
| | 122/127, 31 | | 1 equivalent of acid |

### Example 3: Preparation of benzenesulfonate salt

An appropriate amount (20-30 mg) of compound A was weighed and added to a sample flask at room temperature, and then dissolved or suspended in 0.2 mL of the solvent selected from Table 10. A benzenesulfonic acid solid or a 1 M solution of benzenesulfonic acid in methanol was added for a salt-forming reaction. The solution or suspension was stirred at room temperature for 5-15 h. If no solid is precipitated, an anti-solvent is added to promote precipitation. If there is a solid, the sample is collected by filtration, dried under vacuum at 50 °C for about 3 h, and characterized by XRPD, TGA, DSC, and ¹H-NMR. Specific information and results are listed in Table 10. The XRPD pattern and analysis of a benzenesulfonate crystal form I prepared from group 1 are shown in FIG. 7 and Table 10'.

**Table 10. Preparation of benzenesulfonate salt**

| **Group** | **Solvent** | **Volume of solvent/mass of compound A (mL/g)** | **Acid** | **Anti-solvent (v/vₐₙₜᵢ)** | **Result** |
|---|---|---|---|---|---|
| 1 | EtOH | 6.7 | 14mg | MTBE(1/3) | Benzenesulfonate crystal form I |
| 2 | EA | 6.7 | | NA | Low crystallinity |
| 3 | 2-Me-THF | 6.7 | | | Oil |
| 4 | ACN | 10 | 52 µL | | Benzenesulfonate crystal form I |
| 5 | DCM | 10 | | | Benzenesulfonate crystal form I |

**Table 10'. XRPD analysis of benzenesulfonate crystal form I**

| **2θ/°** | **Relative intensity I/%** | **2θ/°** | **Relative intensity I/%** |
|---|---|---|---|
| 5.464 | 23.0 | 19.438 | 18.3 |
| 5.720 | 14.5 | 19.768 | 32.1 |
| 7.962 | 66.2 | 19.978 | 55.6 |
| 9.001 | 100.0 | 20.489 | 63.6 |
| 9.790 | 40.8 | 21.854 | 8.6 |
| 10.299 | 44.2 | 22.432 | 22.9 |
| 11.980 | 28.0 | 23.299 | 48.7 |
| 12.614 | 31.7 | 23.756 | 20.8 |
| 13.689 | 22.1 | 24.207 | 12.3 |
| 14.071 | 10.7 | 24.613 | 96.9 |
| 14.488 | 44.0 | 25.243 | 46.5 |
| 15.276 | 58.6 | 25.952 | 13.2 |
| 15.802 | 63.3 | 27.014 | 7.3 |
| 16.841 | 18.1 | 27.764 | 14.4 |
| 17.090 | 41.1 | 28.222 | 21.1 |
| 17.298 | 45.7 | 28.656 | 9.2 |
| 17.772 | 12.5 | 30.153 | 8.1 |
| 18.020 | 16.3 | 33.489 | 7.5 |
| 18.204 | 19.0 | 33.684 | 7.2 |
| 18.792 | 10.4 | 34.589 | 5.5 |
| 19.242 | 42.1 | | |

In this example, one crystal form of the benzenesulfonate salt was obtained, which was named as a benzenesulfonate crystal form I. The benzenesulfonate crystal form I was characterized by ¹H-NMR, TGA, and DSC. The relevant characterization results are summarized in Table 11 and FIGs. 8-9. The benzenesulfonate crystal form I had a weight loss of about 3.0% before 117 °C. ¹H-NMR analysis showed that the sample contained 0.4% EtOH and 0.2% MTBE organic solvent, so the TGA weight loss was attributed to the removal of water and organic solvent residues. In the DSC pattern, a broad overlapping endothermic peak with a peak temperature of about 114 °C was attributed to the removal of water (about 1 equivalent of H₂O). The benzenesulfonate crystal form I was determined to be a hydrate.

**Table 11. Solid state characterization results for benzenesulfonate crystal form I**

| **Crystal form solvation** | **DSC, endo Onset/Peak(°C), ΔH (J/g)** | **TGA Wt. loss%/ @T (°C)** | **¹H-NMR** |
|---|---|---|---|
| Benzenesulfonate crystal form I hydrate | 71/114, 68 | 3/RT - 117 | 0.4% EtOH and 0.2% MTBE; 1 equivalent of acid |

### Example 4: Preparation of maleate salt

30 mg of compound A was weighed and added to a sample flask at room temperature, and then dissolved or suspended in 0.2 mL of the solvent selected from Table 12. A maleic acid solid or a 1 M solution of maleic acid in methanol was added for a salt-forming reaction. The solution or suspension was stirred at room temperature for 15 h. If no solid is precipitated, an anti-solvent is added to promote precipitation. If there is a solid, the sample is collected by filtration, and dried under vacuum at 50 °C for about 3 h to give a product, which is characterized by XRPD, TGA, DSC, and ¹H-NMR. Specific information and results are summarized in Table 12. XRPD patterns and analyses of maleate crystal form I and maleate crystal form II prepared from group 2 and group 1 are shown in FIG. 10 and Table 12', and FIG. 11 and Table 12", respectively.

**Table 12. Preparation of maleate salt**

| **Group** | **Solvent** | **Volume of solvent/mass of compound A (mL/g)** | **Acid (mg)** | **Anti-solvent (v/vₐₙₜᵢ)** | **Result** |
|---|---|---|---|---|---|
| 1 | MeOH | 6.7 | 10 | MTBE(1/3) | Maleate crystal form II |
| 2 | EA | 6.7 | | NA | Maleate crystal form I |
| 3 | 2-Me-THF | 6.7 | | | Oil |

**Table 12'. XRPD analysis of maleate crystal form I**

| **2θ/°** | **Relative intensity I/%** | **2θ/°** | **Relative intensity I/%** |
|---|---|---|---|
| 4.221 | 71.4 | 19.216 | 22.3 |
| 7.294 | 81.7 | 20.163 | 12.8 |
| 10.589 | 5.6 | 21.068 | 16.5 |
| 11.193 | 12.6 | 22.223 | 10.4 |
| 12.255 | 24.0 | 22.591 | 23.7 |
| 12.915 | 7.9 | 23.551 | 6.0 |
| 14.687 | 21.1 | 25.083 | 12.4 |
| 15.343 | 20.9 | 26.071 | 100.0 |
| 16.129 | 61.3 | 27.122 | 17.8 |
| 17.194 | 71.0 | 28.171 | 18.5 |
| 18.258 | 7.3 | 30.088 | 9.7 |
| 18.568 | 10.2 | | |

**Table 12". XRPD analysis of maleate crystal form II**

| **2θ/°** | **Relative intensity I/%** | **2θ/°** | **Relative intensity I/%** |
|---|---|---|---|
| 3.959 | 14.1 | 23.851 | 10.9 |
| 7.989 | 100.0 | 24.232 | 15.7 |
| 9.250 | 13.1 | 25.768 | 7.5 |
| 11.194 | 10.2 | 26.766 | 3.7 |
| 13.254 | 11.1 | 27.040 | 9.4 |
| 16.106 | 3.7 | 27.475 | 11.4 |
| 16.643 | 3.3 | 28.315 | 23.4 |
| 18.665 | 5.5 | 29.102 | 3.2 |
| 20.174 | 30.7 | 30.430 | 4.6 |
| 21.487 | 5.0 | 36.377 | 2.5 |
| 22.658 | 8.1 | | |

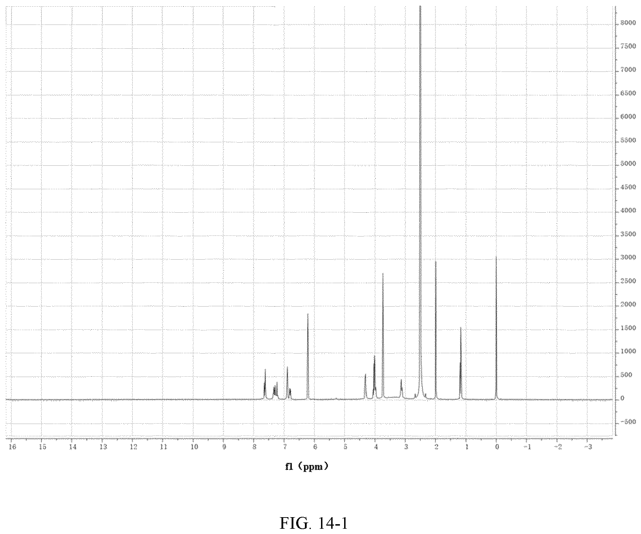

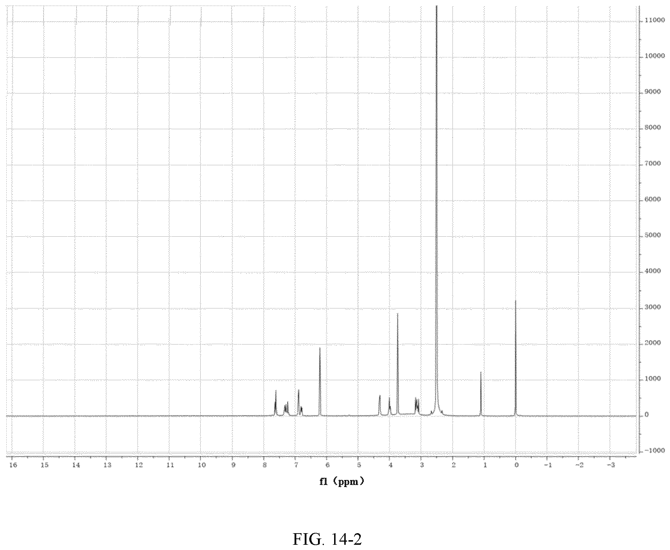

In this example, two crystal forms of the maleate salt were obtained, which were identified and named as maleate crystal forms I and II, respectively. The maleate salt sample was characterized by ¹H-NMR, TGA, and DSC. The relevant characterization results are summarized in Table 13 and FIG. 12, FIG. 13, FIG. 14-1, and FIG. 14-2.

The maleate crystal form I had a weight loss of about 8.4% before 100 °C. ¹H-NMR analysis showed that the sample contained about 13% EA, so the TGA weight loss was attributed to the removal of EA. There was a broad overlapping endothermic peak with a peak temperature of about 83 °C in the DSC pattern, which was attributed to the removal of organic solvent. The maleate crystal form I was determined to be EA solvate.

The maleate crystal form II had a weight loss of about 3.0% before 104 °C. ¹H-NMR analysis showed that the sample contained about 2.0% MTBE, so the TGA weight loss was attributed to the removal of MTBE and water. There was a broad overlapping endothermic peak with a peak temperature of about 108 °C in the DSC pattern, which was attributed to the removal of organic solvent and water.

**Table 13. Solid state characterization results for maleate crystal forms**

| **Crystal form solvation** | **DSC, endo Onset/Peak(°C), ΔH (J/g)** | **TGA Wt. loss%/ @T (°C)** | **¹H-NMR** |
|---|---|---|---|
| Maleate crystal form I EA solvate | 73/83, 57 | 8.4/RT - 100 | 13% EA; 1 equivalent of acid |
| Maleate crystal form II Hydrate/solvate | 94/108, 45 | 3/RT - 104 | 2% MTBE; 1 equivalent of acid |

### Example 5: Preparation of oxalate salt

30 mg of compound A was weighed and added to a sample flask at room temperature, and then dissolved or suspended in 0.2 mL of the solvent selected from Table 14. A 1 M solution of oxalic acid in methanol was added for a salt-forming reaction. The solution or suspension was stirred at room temperature for about 15 h. If no solid is precipitated, an anti-solvent is added to promote precipitation. If there is a solid, the sample is collected by filtration, dried under vacuum at 50 °C for about 5 h, and characterized by XRPD, TGA, DSC, and ¹H-NMR. Specific information and results are listed in Table 14. The XRPD pattern and analysis of an oxalate crystal form I prepared from group 3 are shown in FIG. 15 and Table 14'.

**Table 14. Preparation of oxalate salt**

| **Group** | **Solvent** | **Volume of solvent/mass of compound A (mL/g)** | **Acid (µL)** | **Anti-solvent (v/vₐₙₜᵢ)** | **Result** |
|---|---|---|---|---|---|
| 1 | MeOH | 6.7 | 87 | MTBE(1/3) | Amorphous form |
| 2 | EA | 6.7 | | NA | Oxalate crystal form I |
| 3 | 2-Me-THF | 6.7 | | | Oxalate crystal form I |

**Table 14'. XRPD analysis of oxalate crystal form I**

| **2θ/°** | **Relative intensity I/%** | **2θ/°** | **Relative intensity I/%** |
|---|---|---|---|
| 4.944 | 19.8 | 16.550 | 35.2 |
| 5.261 | 48.2 | 18.638 | 7.5 |
| 7.255 | 19.4 | 19.139 | 7.7 |
| 10.620 | 8.5 | 20.212 | 13.0 |
| 12.244 | 58.7 | 20.949 | 19.6 |
| 12.961 | 6.1 | 24.352 | 7.7 |
| 13.492 | 6.9 | 25.755 | 100.0 |
| 14.172 | 14.0 | 26.503 | 9.7 |
| 14.776 | 15.9 | 27.199 | 6.5 |
| 16.034 | 11.5 | 30.876 | 14.0 |

In this example, one crystal form of the oxalate salt was obtained, which was named as an oxalate crystal form I. The oxalate salt sample prepared from group 3 was characterized by ¹H-NMR, TGA and DSC, and IC. The relevant characterization results are summarized in Tables 15-16 and FIGs. 16-17.

The oxalate crystal form I had a weight loss of about 0.5% before 150 °C. ¹H-NMR analysis showed that there were no organic solvent residues in the sample, so the TGA weight loss was attributed to the removal of adsorbed water. There was a sharp endothermic peak with a peak temperature of about 206 °C in the DSC pattern, which was attributed to the melting of the sample accompanied by decomposition. The IC results showed that the sample had an acid/base ratio of about 1/1. The oxalate crystal form I was determined to be an anhydrate.

**Table 15. Solid state characterization results for oxalate crystal form I**

| **Crystal form solvation** | **DSC, endo Onset/Peak(°C), ΔH (J/g)** | **TGA Wt. loss%/ @T (°C)** | **¹H-NMR** | **IC** |
|---|---|---|---|---|
| Oxalate crystal form I Anhydrous form | 202/206, 168 | 0.5/RT - 150 | No organic solvent residues | 1 equivalent of acid |

**Table 16. IC data for oxalate crystal form I**

| **Sample** | **Mass (mg)** | **Theoretical concentration (µg/mL)** | **IC peak area (µS*min)** | **Calculated C₂O₄²⁻ equivalent** |
|---|---|---|---|---|
| C₂O₄²⁻ standard liquid (C₂O₄²⁻-STD) | NA | 10 | 0.8253 | NA |
| Oxalate crystal form I | 1.30 | 9.7 | 0.8197 | 1 |

### Example 6: Scale-up of oxalate crystal form I

About 300 mg of compound A was weighed and dissolved in 100 µL of MeOH. 78 mg of oxalic acid was added. Then, 2 mL of 2-Me-THF was added. The suspension was stirred for 3 days. The sample was collected by filtration, dried under vacuum at 50 °C for about 15 h, and characterized by XRPD, IC, TGA, DSC, and ¹H-NMR.

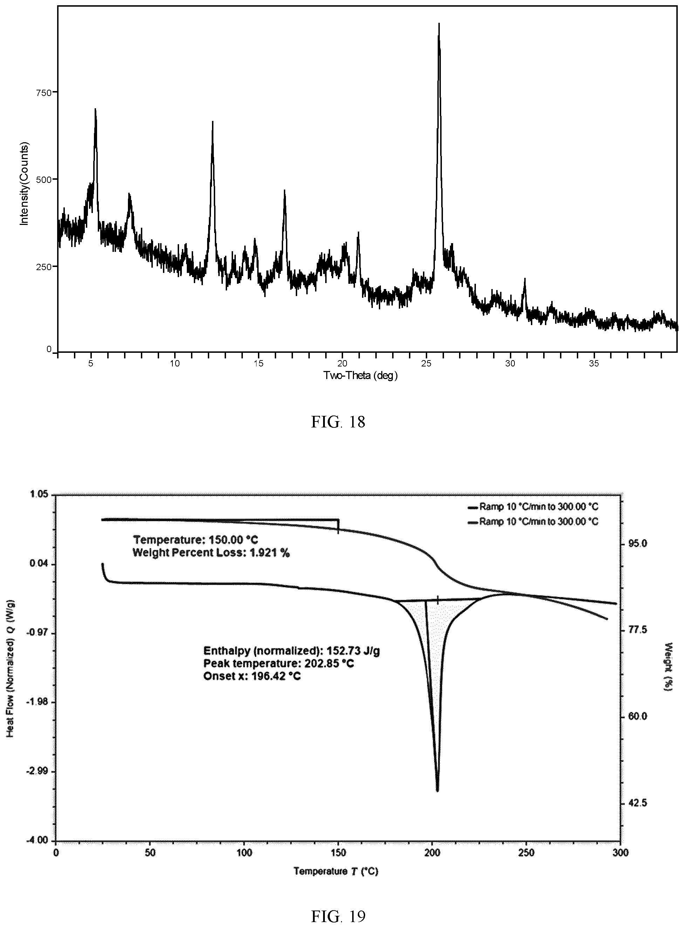

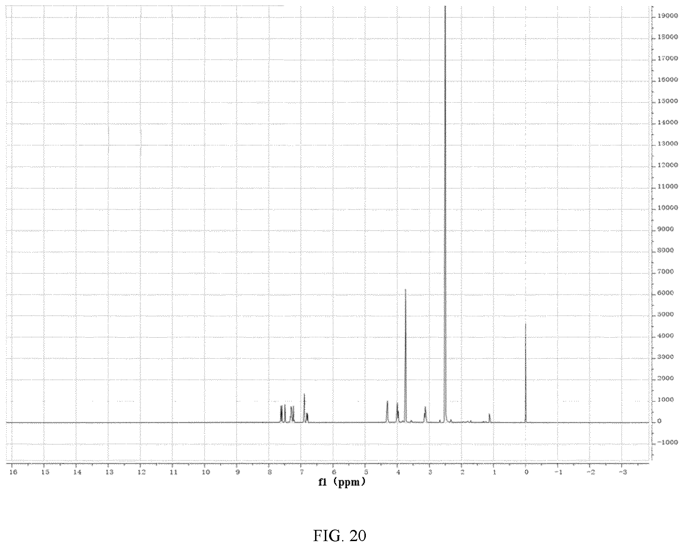

About 200 mg of the oxalate crystal form I was prepared with a yield of about 53%. The sample was analyzed by XRPD, DSC, TGA, DVS, and ¹H-NMR. The IC results showed that the oxalate salt had an acid/base ratio of 1:1. The TGA data showed that the oxalate crystal form I had a weight loss of about 1.9% before 150 °C. The ¹H-NMR analysis showed that the sample contained 2.1% 2-Me-THF. There was an endothermic peak with a peak temperature of 203 °C in the DSC pattern, which was attributed to the melting of the sample accompanied by decomposition. The detailed characterization results are shown in Table 17 and FIGs. 18-20.

**Table 17. Characterization results for oxalate crystal form I**

| **DSC, endo Onset/Peak(°C), ΔH (J/g)** | **TGA Wt. loss%/ @T (°C)** | **DVS (Wt. gain%, 80/90%R)** | **¹H-NMR** |
|---|---|---|---|
| 196/203, 153 | 1.9/RT-150 | 2.3/3.1 | 2.1% 2-Me-THF |

### Example 7: Preparation of fumarate salt and crystal form I thereof

An appropriate amount of compound A was weighed and added to a sample flask at room temperature, and then dissolved or suspended in 0.2 mL of the solvent selected from Table 18. A fumaric acid solid was added for a salt-forming reaction. The solution or suspension was stirred at room temperature for a certain period of time (about 3-15 h). If no solid is precipitated, an anti-solvent is added to promote precipitation. If there is a solid, the sample is collected by filtration, dried under vacuum at 50 °C for about 5 h, and characterized by XRPD, TGA, DSC, and ¹H-NMR. Specific information and results are listed in Table 18.

**Table 18. Preparation of fumarate salt**

| **Group** | **Solvent** | **Volume of solvent/mass of compound A (mL/g)** | **Molar ratio of acid to compound A** | **Result** |
|---|---|---|---|---|
| 1 | MeOH | 6.7 | 1.1 | Fumarate crystal form I |
| 2 | EA | 6.7 | | Fumarate crystal form I + fumaric acid |
| 3 | 2-Me-THF | 6.7 | | Oil |
| 4 | EA/MeOH (v/v, 60:1) | 4.5 | 0.55 | Fumarate crystal form I |
| 5 | EA | 10 | | Fumarate crystal form I |

The XRPD and analytical pattern of the fumarate crystal form product prepared from group 4 are shown in FIG. 21 and Table 19'. The fumarate crystal form I was characterized by ¹H-NMR, TGA, and DSC. The relevant characterization results are summarized in Table 19 and FIGs. 22-23.

The fumarate crystal form I had almost no weight loss before 150 °C. ¹H-NMR analysis showed that the sample contained 0.5 equivalents of fumaric acid (i.e., the fumarate salt was the salt of compound A and fumaric acid in a molar ratio of 1:0.5). There was a sharp endothermic peak with a peak temperature of about 157 °C in the DSC pattern, which was attributed to the melting of the sample accompanied by decomposition. The fumarate crystal form I was determined to be an anhydrate.

**Table 19'. XRPD analysis of fumarate crystal form I**

| **2θ/°** | **Relative intensity I/%** | **2θ/°** | **Relative intensity I/%** |
|---|---|---|---|
| 3.907 | 43.3 | 21.396 | 21.4 |
| 10.456 | 21.5 | 22.579 | 3.1 |
| 11.690 | 14.9 | 23.680 | 38.8 |
| 12.217 | 6.7 | 25.168 | 6.6 |
| 13.938 | 84.0 | 25.645 | 2.9 |
| 15.776 | 8.8 | 26.372 | 100.0 |
| 16.866 | 40.0 | 27.409 | 32.4 |
| 17.732 | 20.1 | 27.870 | 24.5 |
| 18.612 | 7.9 | 29.707 | 6.9 |
| 19.015 | 5.6 | 31.243 | 9.5 |
| 19.673 | 2.0 | 32.308 | 4.2 |
| 20.202 | 3.6 | 34.593 | 7.7 |
| 20.686 | 3.0 | 36.139 | 5.1 |

**Table 19. Solid state characterization results for fumarate crystal form I**

| **Crystal form solvation** | **DSC, endo Onset/Peak(°C), ΔH (J/g)** | **TGA Wt. loss%/ @T (°C)** | **¹H-NMR** |
|---|---|---|---|
| Fumarate crystal form I Anhydrous form | 155/157, 127 | 0/RT - 150 | 0.5 equivalents of acid |

### Example 8: Preparation of fumarate crystal form II

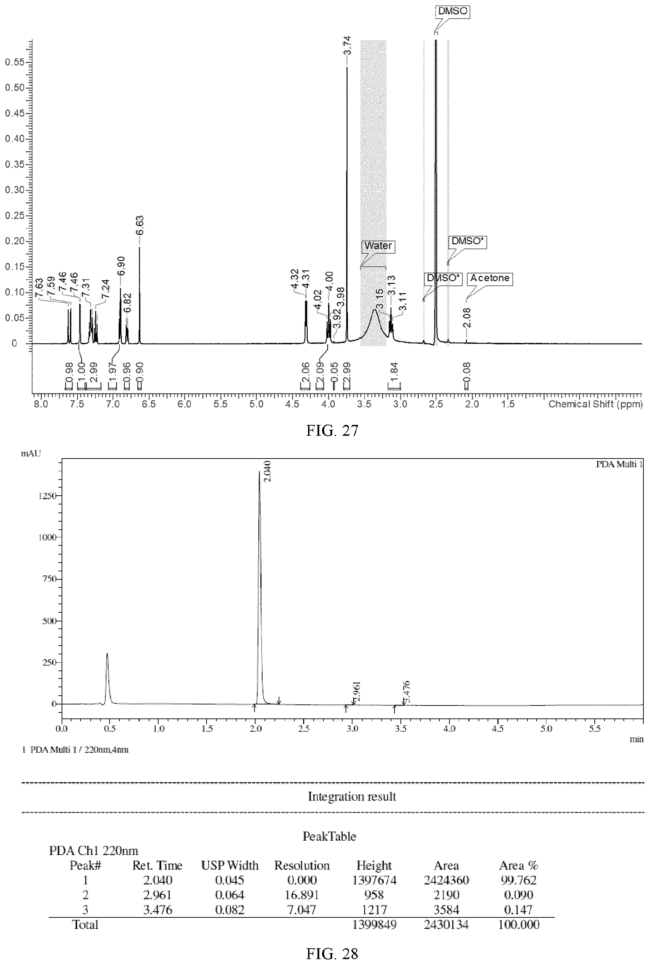

6.56 g of crude compound A was taken, and 46 mL of a mixed solvent ACN and THF in a volume ratio of 2:1 was added. After the resulting mixture was heated to 60-70 °C for refluxing, the solid showed no dissolution. Then, 1.10 g (0.55 eq) of fumaric acid was added, and the solid remained undissolved. The mixture was stirred at 60-70°C for 1 h, naturally cooled to 10-20 °C, stirred for 16 h, and filtered. The filter cake was concentrated by rotary evaporation to give a yellow solid as a fumarate salt (7.32 g). The fumarate salt was characterized by XRPD, TGA, DSC, and ¹H-NMR. The relevant characterization results are summarized in Table 20 and FIGs. 24-27. The molar ratio of fumaric acid to compound A was 0.5:1. The fumarate salt was in an anhydrous crystal form, which had a chemical purity of 99.3%, had high crystallinity, and was named as a fumarate crystal form II.

The PLM result showed that the fumarate crystal form II obtained in this example consisted of particles with irregular morphology of 10-80 µm. The PSD result showed that the fumarate crystal form II had Dv (10) of 8.52 µm and Dv (90) of 43.2 µm.

**Table 20. Solid state characterization results for fumarate crystal form II**

| **Crystal form solvation** | **DSC, endo Onset/Peak(°C), ΔH (J/g)** | **TGA Wt. loss%/ @T (°C)** | **¹H-NMR** |
|---|---|---|---|
| Fumarate crystal form II Anhydrous form | 180/181, 143 | 0.1/RT - 150 | 0.5 equivalents of acid |

**Table 20'. XRPD analysis of fumarate crystal form II**

| **2θ/°** | **Relative intensity I/%** | **2θ/°** | **Relative intensity I/%** |
|---|---|---|---|
| 8.603 | 2.8 | 23.157 | 3.5 |
| 10.039 | 4.7 | 23.859 | 3.8 |
| 11.441 | 28.9 | 24.601 | 30.5 |
| 11.841 | 8.1 | 25.199 | 92.5 |
| 12.241 | 16.4 | 25.923 | 4.4 |
| 12.639 | 14.3 | 26.279 | 4.3 |
| 13.741 | 25.3 | 27.539 | 45.2 |
| 14.698 | 3.2 | 28.779 | 22.6 |
| 15.842 | 9.7 | 29.720 | 3.6 |
| 16.099 | 15.3 | 30.560 | 8.0 |
| 17.119 | 10.0 | 31.221 | 5.4 |
| 17.540 | 3.8 | 33.139 | 5.5 |
| 18.440 | 4.9 | 33.921 | 7.6 |
| 19.060 | 12.6 | 34.684 | 2.9 |
| 19.681 | 4.7 | 35.362 | 2.2 |
| 20.059 | 4.7 | 36.481 | 2.2 |
| 20.803 | 6.1 | 37.260 | 4.3 |
| 21.202 | 11.9 | 38.380 | 3.3 |
| 22.059 | 100.0 | 39.122 | 3.2 |
| 22.499 | 45.1 | | |

Other examples for the preparation of fumarate crystal form II are shown in Table 20-1.

**Table 20-1**

| Group | Salt-forming system (v represents the ratio of the volume mL of the solvent to the mass g of compound A in the salt-forming system) | Operation | Yield | Result | Purity (HPLC test) |
|---|---|---|---|---|---|
| 1 | MeOH:THF:A CN (v = 24, volume ratio of 4:10:10) | 5.0 g of compound A was taken, and 70 mL of a mixed solvent of MeOH and THF in a volume ratio of 4:10 was added. After the resulting mixture was heated to 60-70 °C for refluxing, the solid showed no dissolution. Then, 1.14 g of fumaric acid was added for complete dissolution of the system. The system was stirred at 60-70 °C for 1 h, and naturally cooled to 20-30 °C. 50 mL of ACN was added, and the resulting mixture was stirred until a solid was precipitated, and then stirred for 12 h until a large amount of solid was precipitated. The mixture was filtered. The filter cake was dried using a rotary evaporator. | 55% | XRPD: Fumarate crystal form II | 99.76% (FIG. 28) |
| 2 | MeOH:THF:A CN (v = 24, volume ratio of 10:4:10) | 3.0 g of compound A was taken, and 52 mL of a mixed solvent of MeOH and THF in a volume ratio of 10:4 was added. After the resulting mixture was heated to 60-70 °C, the solid showed no dissolution. Then, 685 mg of fumaric acid was added, and the solid was gradually dissolved until the complete dissolution of the system. The system was naturally cooled to 10-20 °C, and no solid was precipitated. 30 mL (10 v) of ACN was added, and the resulting mixture was stirred for 1 h until a solid was slowly precipitated, and stirred for another 12 h at 10-20 °C. | 45% | XRPD: Fumarate crystal form II | 99.83% |
| 3 | MeOH:THF:A CN (v = 17, volume ratio of 3:5:9) | 5.0 g of compound A was taken, and 40 mL of a mixed solvent of MeOH and THF in a volume ratio of 3:5 was added. After the resulting mixture was heated to 60-70 °C for refluxing, 1.14 g of fumaric acid was added, and the solid was gradually dissolved until the complete dissolution of the system. The system was stirred at 60-70 °C for 1 h, and naturally cooled to 40-50 °C. 45 mL of ACN was added, and the resulting mixture was cooled to 10-20 °C, stirred for 30 min until a large amount of solid was precipitated, and stirred at 10-20 °C for another 12 h. | 65% | XRPD: Fumarate crystal form II | 99.72% |
| 4 | EA:MeOH (v = 36, volume ratio of 35:1) | 2.0 g of compound A was taken, and 72 mL of a mixed solvent of EA and MeOH in a volume ratio of 35:1 was added. The resulting mixture was heated to 20-30 °C. 457 mg of fumaric acid was added, and the resulting mixture was stirred at 20-30 °C for 12 h and filtered. The filter cake was dried using a rotary evaporator. (Complete dissolution was not achieved in the salt-forming process) | 87% | XRPD: Fumarate crystal form II | 99.62% (FIG. 29) |
| 5 | MeOH:THF:A CN (v = 17, volume ratio of 3:6:15) | 10 g of compound A was taken, and 170 mL of a mixed solvent of MeOH, THF, and ACN in a volume ratio of 3:6:8 was added. The resulting mixture was heated to 60-70 °C. 3.00 g of fumaric acid was added, and the solid was gradually dissolved until the complete dissolution of the system. 70 mL of ACN was added, and the resulting mixture was stirred at 60-70 °C for 1 h, and naturally cooled to 53 °C. A crystal seed of the fumarate crystal form II^{[1]} was added, and the resulting mixture was cooled to 10-20 °C, with a large amount of solid precipitated in the cooling process, stirred for 12 h at 10-20 °C, and then filtered. The filter cake was concentrated by rotary evaporation. | 80% | XRPD: Fumarate crystal form II | N/A |

| | | | | | |
|---|---|---|---|---|---|
| Note: [1] it can be understood by those skilled in the art that the added crystal seed of fumarate crystal form II is the prepared fumarate crystal form II, e.g., the fumarate crystal form II obtained with reference to groups 1 to 4 of Table 20. | | | | | |

### Example 9: Fumarate crystal form I - scale-up preparation

About 300 mg of compound A and 50.21 mg of fumaric acid were weighed and dispersed in 4.5 mL of a mixed solvent (EA/MeOH, volume ratio of 35/1). Then, the mixture was left to stand at room temperature and stirred overnight (about 15 h). The sample was collected by filtration and dried under vacuum at 50 °C for about 3 h to give the fumarate crystal form I (about 315 mg, yield: 90%). The sample was analyzed by XRPD, DSC, TGA, DVS, and ¹H-NMR.

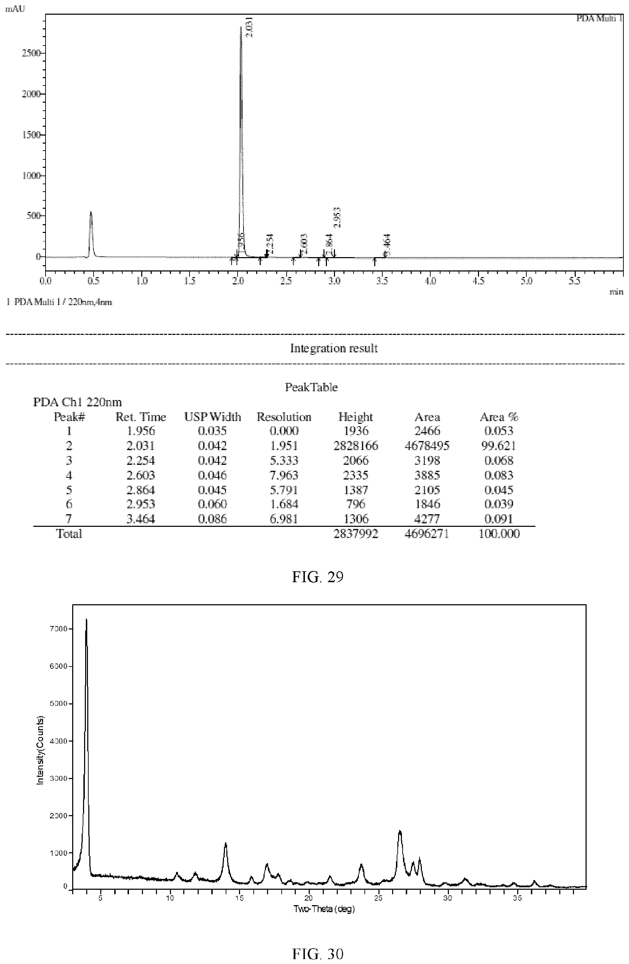

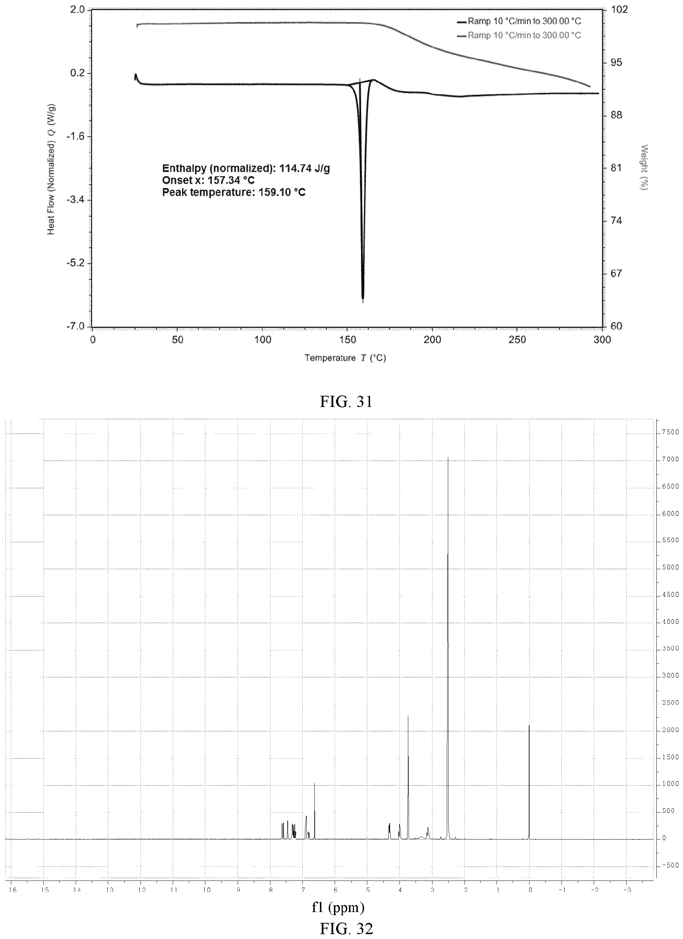

The TGA data showed that the fumarate crystal form I had almost no weight loss before 100 °C. The ¹H-NMR analysis showed that the sample had no organic solvent residues. There was an endothermic peak with a peak temperature of 159 °C in the DSC pattern, which was attributed to the melting of the sample accompanied by decomposition. The detailed characterization results are shown in Table 21 and FIGs. 30-32.

**Table 21. Characterization results for fumarate crystal form I**

| **Sample Batch #** | **DSC, endo Onset/Peak(°C), ΔH (J/g)** | **TGA Wt. loss%/ @T (°C)** | **DVS (Wt. gain%, 80/90%R)** | **¹H-NMR** |
|---|---|---|---|---|
| Fumarate crystal form I | 157/159, 115 | 0/RT - 100 | <0.2 | No organic solvent residues; 0.5 equivalents of acid |

### Example 10: Preparation of tartrate salt

30 mg of compound A was weighed and added to a sample flask at room temperature, and then dissolved or suspended in 0.2 mL of the solvent selected from Table 22. An L-tartaric acid solid was added for a salt-forming reaction. The solution or suspension was stirred at room temperature overnight (about 15 h). If no solid is precipitated, an anti-solvent is added to promote precipitation. If there is a solid, the sample is collected by filtration, dried under vacuum at 50 °C for about 3 h, and characterized by XRPD, TGA, DSC, and ¹H-NMR. Specific information and results are listed in Table 22. The XRPD pattern and analysis of the tartrate salt prepared from group 1 are shown in FIG. 33 and Table 22'.

**Table 22. Preparation of tartrate salt**

| **Group** | **Solvent** | **Volume of solvent/mass of compound A (mL/g)** | **Acid (mg)** | **Anti-solvent (v/vₐₙₜᵢ)** | **Result** |
|---|---|---|---|---|---|
| 1 | MeOH | 6.7 | 13 | MTBE(1/3) | Tartrate crystal form I |
| 2 | EA | 6.7 | | NA | Tartrate crystal form I |

**Table 22'. XRPD analysis of tartrate crystal form I**

| **2θ/°** | **Relative intensity** | **2θ/°** | **Relative** |
|---|---|---|---|
| | **I/%** | | **intensity I/%** |
| 8.909 | 11.5 | 23.969 | 11.4 |
| 11.403 | 15.0 | 24.915 | 24.6 |
| 13.386 | 69.7 | 25.584 | 100.0 |
| 15.251 | 10.1 | 26.084 | 43.3 |
| 16.983 | 80.8 | 26.727 | 57.9 |
| 17.274 | 39.9 | 27.565 | 18.4 |
| 17.850 | 71.6 | 28.343 | 8.6 |
| 18.297 | 24.5 | 29.276 | 6.0 |
| 18.534 | 63.8 | 31.571 | 11.8 |
| 19.095 | 8.5 | 33.639 | 4.5 |
| 19.662 | 89.7 | 34.290 | 13.3 |
| 20.462 | 40.4 | 35.696 | 10.8 |
| 22.277 | 10.1 | 37.178 | 9.6 |
| 22.866 | 46.8 | | |

In this example, one crystal form of the tartrate salt was obtained, which was named as a tartrate crystal form I. The tartrate salt sample was characterized by ¹H-NMR, TGA, and DSC. The relevant characterization results are summarized in Table 23 and FIGs. 34-35.

The tartrate crystal form I had only a 0.8% weight loss before 150 °C. ¹H-NMR analysis showed that the sample contained 0.8% MTBE and 1 equivalent of acid. There was a sharp endothermic peak with a peak temperature of about 135 °C in the DSC pattern, which was attributed to the melting of the sample. The tartrate crystal form I was determined to be an anhydrate.

**Table 23. Solid state characterization results for tartrate crystal form I**

| **Crystal form solvation** | **DSC, endo Onset/Peak(°C), ΔH (J/g)** | **TGA Wt. loss%/ @T (°C)** | **¹H-NMR** |
|---|---|---|---|
| Tartrate crystal form I Anhydrous form | 127/135, 54 | 0.8/RT - 150 | 0.8% MTBE; 1 equivalent of acid |

### Example 11: Preparation of free base crystal form I

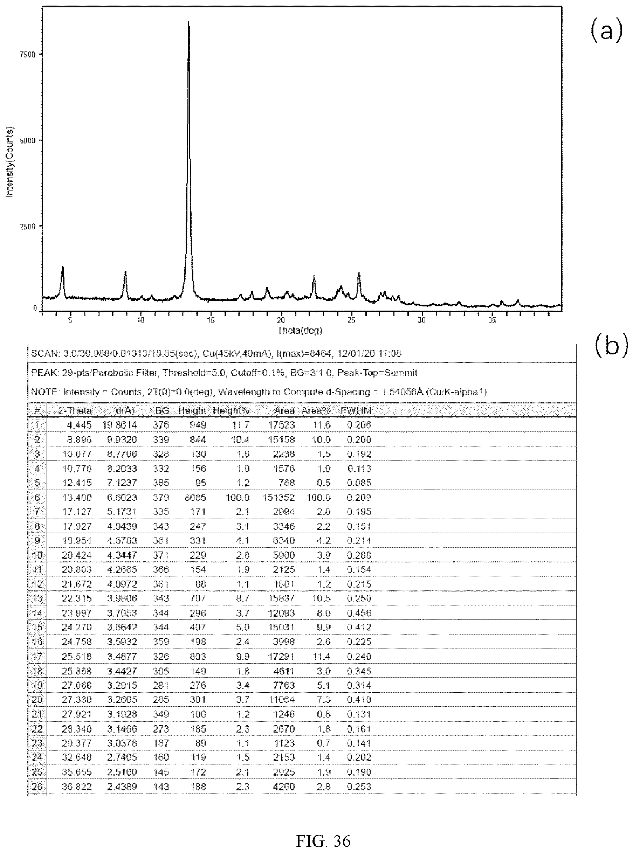

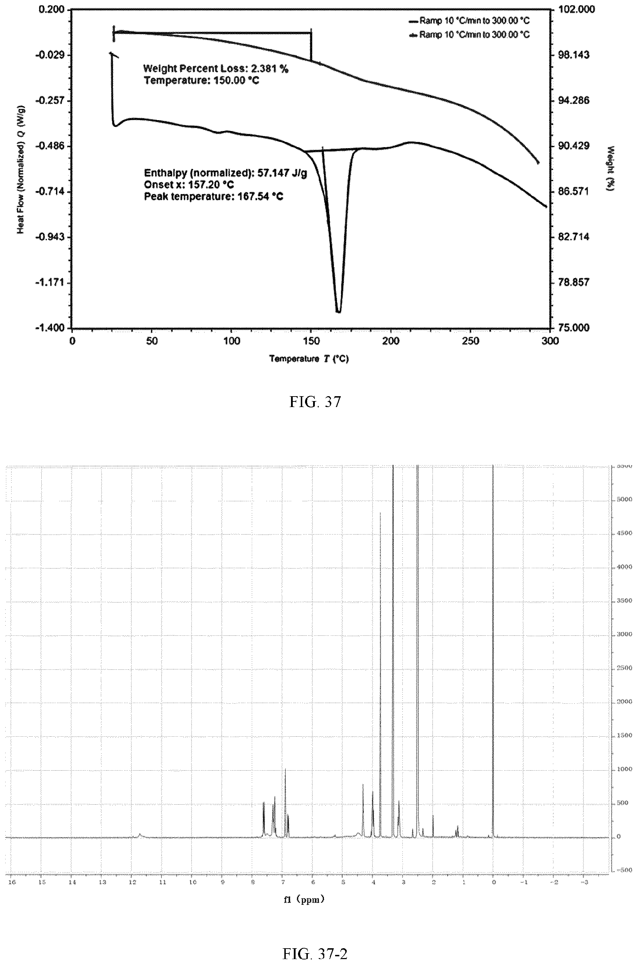

30 mg of compound A was taken, and 0.2 mL of ethyl acetate was added to obtain a suspension, which was then stirred at room temperature for 15 h and filtered. The sample collected by filtration was dried at 50 °C for 3 h in vacuum to give a free base crystal form I. The XRPD detection pattern and analysis are shown in FIG. 36. The DSC-TGA detection results are shown in FIG. 37; DSC: 157 °C. The ¹H-NMR spectrum is shown in FIG. 37-2.

**Table 24. Solid state characterization results for free base crystal form I**

| **Crystal form solvation** | **DSC, endo Onset/Peak(°C), ΔH (J/g)** | **TGA Wt. loss%/ @T (°C)** | **Note** |
|---|---|---|---|
| Free base crystal form I Anhydrate | 157/168, 57 | 2.4/RT - 150 | 1.9% EA |

### Example 12: Preparation of free base crystal form II

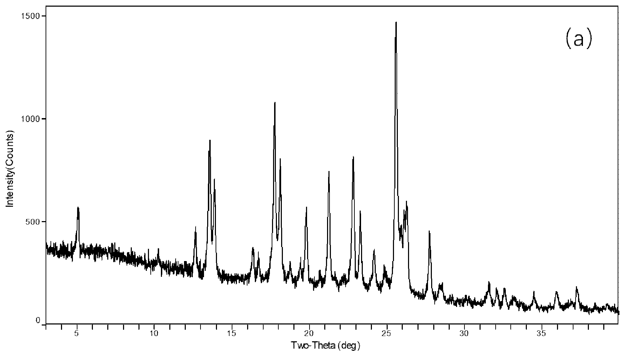

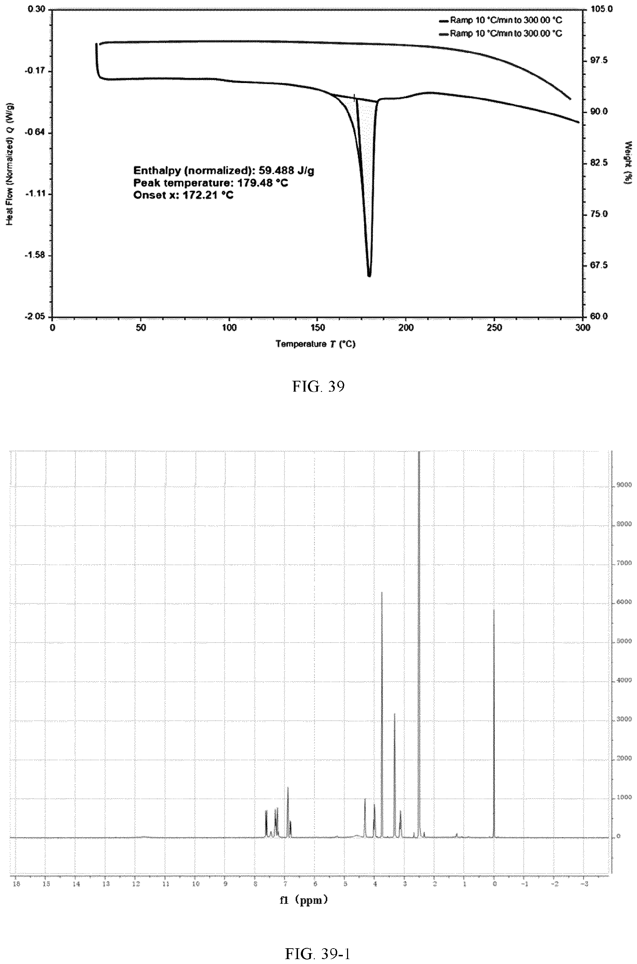

300 mg of compound A was taken and dispersed in 2 mL of a mixed solvent (EtOH/Water, v/v = 1/10). The dispersion was stirred at room temperature for 3 days and filtered. The sample collected by filtration was dried at 50 °C for about 15 h in vacuum to give a free base crystal form II. The XRPD detection pattern and analysis of the obtained crystal form II are shown in FIG. 38. The DSC-TGA detection result is shown in FIG. 39. The ¹H-NMR spectrum is shown in FIG. 39-1.

**Table 25. Solid state characterization results for free base crystal form II**

| **Crystal form solvation** | **DSC, endo Onset/Peak(°C), ΔH (J/g)** | **TGA Wt. loss%/ @T (°C)** | **Note** |
|---|---|---|---|
| Free base crystal form II Anhydrate | 172/179, 59 | 0/RT - 150 | No EtOH residue High melting point |

### Example 13: Hygroscopicity test of free base crystal form II, oxalate crystal form I, fumarate crystal form I, and fumarate crystal form II

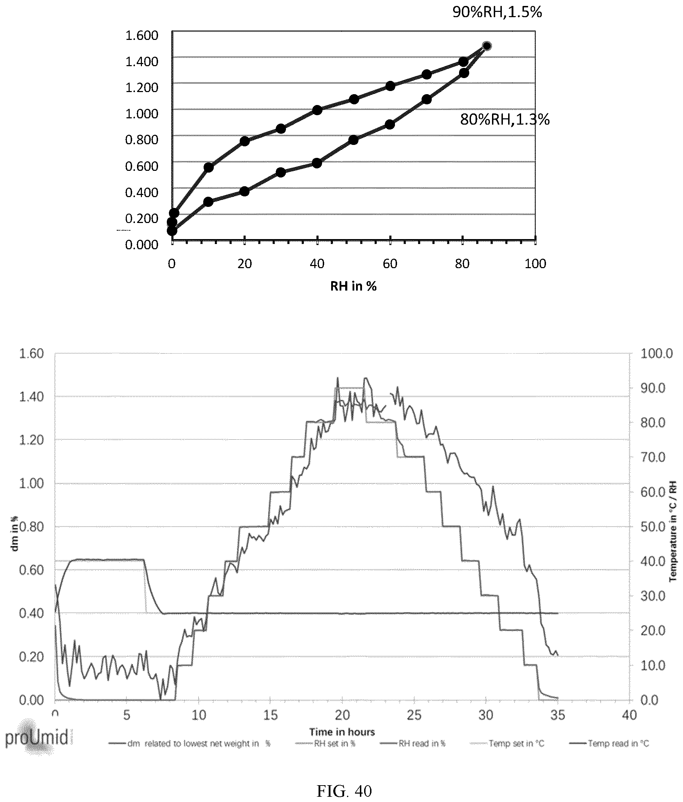

About 40 mg of each of the free base crystal form II, oxalate crystal form I, fumarate crystal form I, and fumarate crystal form II was weighed, placed in a tared DVS tray, and evaluated for hygroscopicity according to the dynamic vapor sorption (DVS) analysis method described above. The DVS data showed that the free base crystal form II had a 1.3/1.5% weight gain over the range of 0.0% RH to 80/90% RH, and was slightly hygroscopic. The detailed characterization results are shown in FIG. 40. The XRPD pattern of the free base crystal form II did not change before and after the DVS test, and the crystal form remained unchanged.

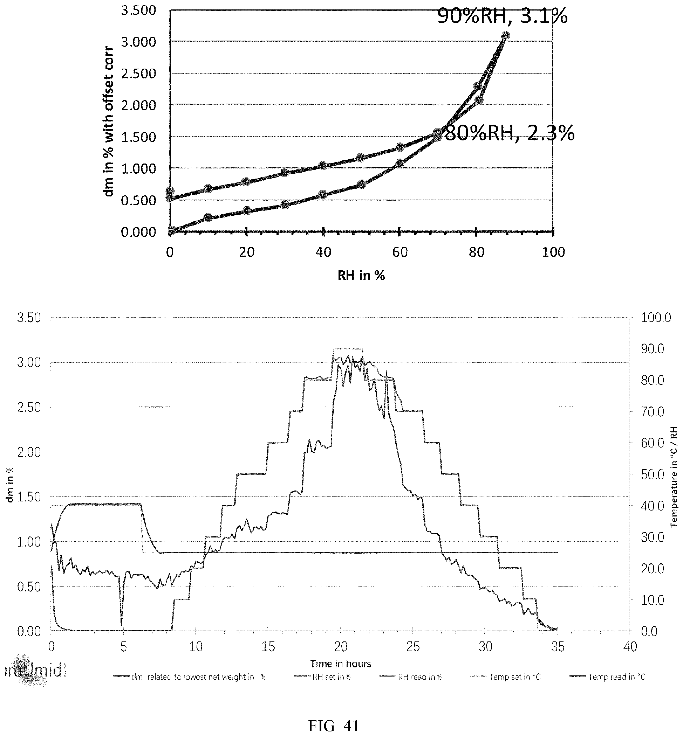

The DVS data showed that the oxalate crystal form I had a 2.3/3.1% weight gain over the range of 0.0% RH to 80/90% RH, and the sample was slightly hygroscopic. The detailed characterization results are shown in FIG. 41. The XRPD pattern of the oxalate crystal form I was kept consistent before and after the DVS test, and the crystal form remained unchanged.

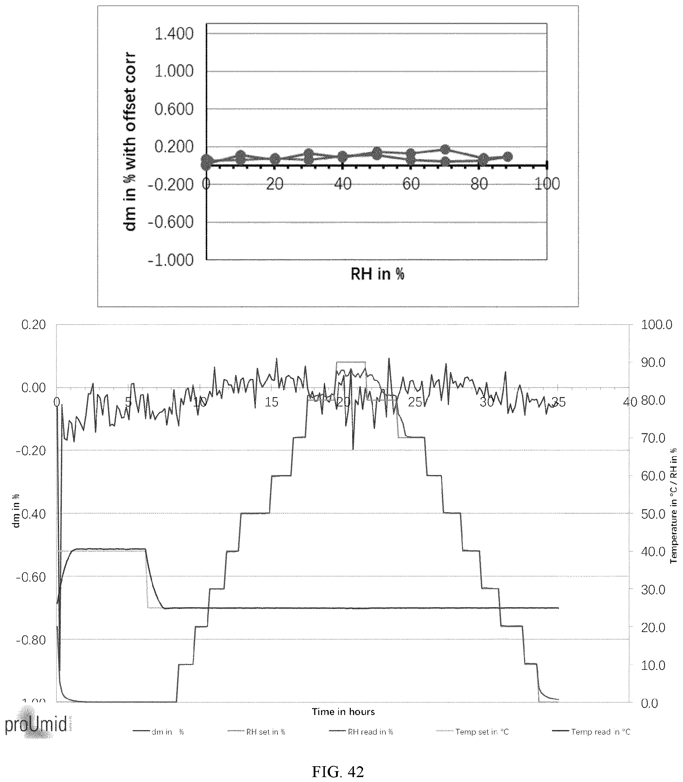

The DVS data showed that the fumarate crystal form I had a weight gain <0.2% over the range of 0.0% RH to 80/90% RH, and was non-hygroscopic. The detailed characterization results are shown in FIG. 42. The XRPD pattern of the fumarate crystal form I was kept consistent before and after the DVS test, and the crystal form remained unchanged.

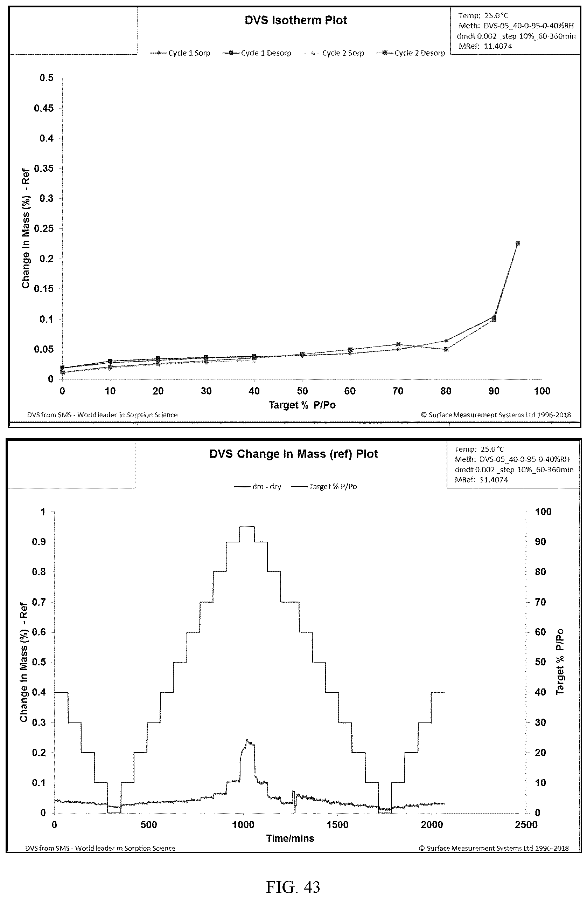

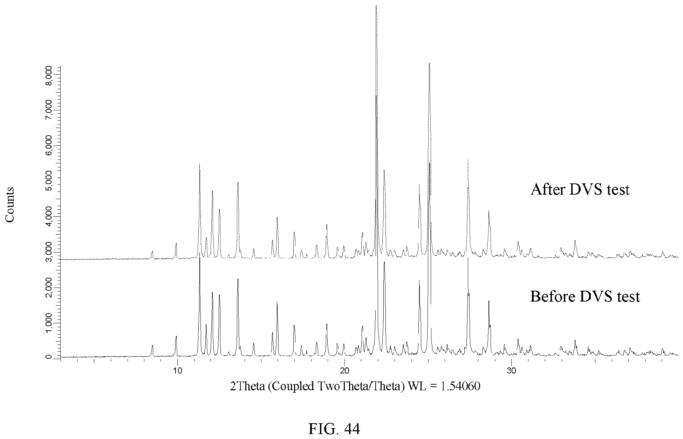

The DVS data showed that the fumarate crystal form II absorbed about 0.2% water from 0.0% RH to 95% RH, and was non-hygroscopic. The XRPD pattern of the fumarate crystal form II was kept consistent before and after the DVS test, and the crystal form remained unchanged. The detailed characterization results are shown in FIGs. 43 and 44.

### Example 14: Solubility test of free base crystal form II, oxalate crystal form I, fumarate crystal form I, and fumarate crystal form II

The solubility of the free base crystal form II, oxalate crystal form I, fumarate crystal form I, and fumarate crystal form II was determined in a biologically relevant media at 37 °C.

15 mg of each of free base crystal form II, oxalate crystal form I, fumarate crystal form I, and fumarate crystal form II was weighed and dispersed in 5.0 mL of a biologically relevant medium. The suspension was incubated by shaking on a shaker at 100 rpm at 37 °C. 1 mL of the dispersion was taken at 0.5 h, 2 h, and 24 h, and filtered. The filtrate was tested for the solubility by HPLC and for the pH value by a pH meter. The filter cake was characterized for the crystal form by XRPD. The relevant characterization results are summarized in Table 26.

**Table 26. Results of biologically relevant media**

| **Sample** | **Medium** | **Solubility (µg/mL)** | | | **XRPD** | **pH** | |
|---|---|---|---|---|---|---|---|
| | | **0.5h** | **2h** | **24h** | | **0h** | **24h** |
| Free base crystal form II | FaSSIF | 8.24 | 5.14 | 6.44 | Remains unchanged | 6.50 | 6.49 |
| | FeSSIF | 33.38 | 42.87 | 48.68 | Remains unchanged | 5.00 | 4.99 |
| | SGF | 1010 | 1027 | 1167 | Remains unchanged | 1.20 | 1.29 |
| Oxalate crystal form I | FaSSIF | 19.34 | 12.35 | 13.41 | Oxalate crystal form I + free base crystal form II (trace) | 6.50 | 5.94 |
| | FeSSIF | 96.23 | 194.1 | 255.4 | Remains unchanged | 5.00 | 4.94 |
| | SGF | 73.75 | 123.4 | 139.9 | Remains unchanged | 1.20 | 1.24 |
| Fumarate crystal form I | FaSSIF | 12.19 | 3.57 | 4.25 | Free base crystal form II | 6.50 | 5.96 |
| | FeSSIF | 109.2 | 75.30 | 61.39 | Free base crystal form II | 5.00 | 4.90 |
| | SGF | 1899 | 2135 | 2146 | Trace solid | 1.20 | 1.29 |
| Fumarate crystal form II | FaSSIF | - | - | 0.28 | Mixed crystal of free base crystal form I and free base crystal form II | 6.50 | 6.2 |
| | FeSSIF | - | - | 132.92 | Mixed crystal of form II, free base crystal form I and free base crystal form II | 5.00 | 4.9 |
| | SGF | - | - | 1425.24 | Remains unchanged | 1.20 | 1.2 |

Among three biologically relevant media, the fumarate crystal form I had a higher solubility than the free base crystal form II (0.5 h). The two solid forms had the highest solubility (1.0 mg/mL vs 1.9 mg/mL) in the SGF medium at 0.5 h, which was almost 10 times more than that of the oxalate crystal form I (0.07 mg/mL). Both salt forms showed dissociation into the free base during the solubility test. In the solubility test, the free base crystal form II remained unchanged at 24 h in the biologically relevant media. The oxalate crystal form I remained unchanged at 24 h in FeSSIF and SGF, but partially dissociated into the free base crystal form II at 24 h in FaSSIF. The fumarate crystal form I dissociated into the free base crystal form II at 0.5 h in FaSSIF and FeSSIF media.

The solubility test results showed that the solubility of the fumarate crystal form I was about twice that of the free base crystal form II at 0.5 h in all three biologically relevant media.

### Example 15: Solid state stability test of free base crystal form II, oxalate crystal form I, fumarate crystal form I, and fumarate crystal form II

An appropriate amount of each of the free base crystal form II, oxalate crystal form I, fumarate crystal form I, and fumarate crystal form II was weighed and stored under two conditions of 60 °C/sealed and 40 °C/75% RH open for 1 week and 2 weeks. The samples after being stored 0 days, 1 week, and 2 weeks were taken and each dissolved in a diluent to prepare about 1.0 mg/mL solution, which was tested for chemical stability by HPLC. The solid sample after being stored for 1 week or 2 weeks was tested for physical stability by XRPD. The relevant characterization results are summarized in Tables 27-28.

**Table 27. Stability evaluation result (7 days)**

| **Sample** | **Purity - 0 weeks and 1 week (Area%) 265 nm** | | | **XRPD - 1 week** | |
|---|---|---|---|---|---|
| | **Initial purity** | **40 °C/75%RH** | **60 °C/sealed** | **40 °C/75%RH** | **60 °C/sealed** |
| Free base crystal form II | 98.62 | 98.62 | 98.64 | Remains unchanged | Remains unchanged |
| Oxalate crystal form I | 96.97 | 96.97 | 96.13 | Remains unchanged | Remains unchanged |
| Fumarate crystal form I | 99.85 | 99.85 | 99.86 | Remains unchanged | Remains unchanged |
| Fumarate crystal form II | 99.30 | 99.20 | 99.20 | Remains unchanged | Remains unchanged |

**Table 28. Stability evaluation results (14 days)**

| **Sample** | **Purity - 0 weeks and 2 weeks (Area%) 265 nm** | | | **XRPD - 2 weeks** | |
|---|---|---|---|---|---|
| | **Initial purity** | **40 °C/75%RH** | **60 °C/sealed** | **40 °C/75%RH** | **60 °C/sealed** |
| Free base crystal form II | 99.83 | 99.83 | 99.83 | Remains unchanged | Remains unchanged |
| Fumarate crystal form I | 99.73 | 99.74 | 99.75 | Remains unchanged | Remains unchanged |

The stability results showed that the free base crystal form II and the fumarate crystal form I were physically and chemically stable at one week under the two conditions of 60 °C/sealed and 40 °C/75% RH open, but the oxalate was degraded.

The 14-day stability test results showed that the free base crystal form II and the fumarate crystal form I were physically and chemically stable at two weeks under the two conditions of 60 °C/sealed and 40 °C/75% RH open.

### Example 16: Comparison of pharmacokinetic study experiments of free base crystal form II and fumarate crystal form I

In the experiment, 12 SD rats (purchased from Beijing Vital River Laboratory Animal Technology Co., Ltd.) weighing 180-280 g, half male and female, were used. The animals were randomly divided into four groups of 3 animals each, with female rats in the first and third groups and male rats in the second and fourth groups.

The free base crystal form II and the fumarate crystal form I of compound A were each dissolved with 5% TPGS to obtain a solution at a drug concentration of 20 mg/mL (based on compound A), which was then administered intragastrically in a volume of 10 mL/kg at a dose of 20 mg/kg with a frequency of QD (once a day). Blood was collected from the orbital venous plexus of rats at various time points (0.167 h, 0.5 h, 1 h, 2 h, 3 h, 4 h, 6 h, 9 h, 12 h, and 24 h) after administration. The concentration of compound A in the plasma was determined.

Data will be analyzed by a non-compartmental model using WinNonlin (version 5.2.1 Pharsight, Mountain View, CA) to obtain PK parameters (parameters selected for different routes of administration, such as C₀, Cₘₐₓ, Tₘₐₓ, AUC₀₋ₗₐₛₜ, AUC_{inf}, T_{1/2}, CL, and Vz).

The pharmacokinetic parameters are shown in Table 29.

**Table 29**

| **Compound/group** | **Cₘₐₓ (ng/mL)** | **Tₘₐₓ (h)** | **T_{1/2} (h)** | **AUCₗₐₛₜ (ng/mL*h)** | **AUC_{inf} (ng/mL*h)** |
|---|---|---|---|---|---|
| First group - free base crystal form II | 3617±321 | 0.67±0.17 | 2.88 ±0.46 | 14778±3318 | 18164±150 |
| Second group - free base crystal form II | 3297±676 | 1.00±0.00 | 3.33±0.40 | 19604±2132 | 19790±2233 |
| Third group - fumarate crystal form I | 6357±342 | 0.83±0.17 | 3.01±0.22 | 32884±1318 | 33018±1303 |
| Fourth group - fumarate crystal form I | 3053±198 | 0.67±0.17 | 3.44±0.29 | 23896±2934 | 24142±2905 |

From the comparison of pharmacokinetic parameters of the free base and the fumarate salt of the compound A administered intragastrically at the same dose in female and male rats, it could be seen that the exposure of compound A in rats after the intragastric administration of the fumarate salt of compound A to female and male rats was 2.23 times and 1.22 times that of compound A in rats after the intragastric administration of the free base to female and male rats.

The acid addition salts of compound A and the crystal forms of the salts provided by the present disclosure have the characteristics of high solubility, good stability, high purity, few impurities and high bioequivalence, and are beneficial to storage, quality control, and druggability.

The present disclosure provides an acid addition salt of compound A, a crystal form of the salt, and a preparation method therefor. The preparation method features simplicity of the process, ease of implementation, mild conditions for reaction, and high product yields. Moreover, multiple purification processes are not necessary, and the operation is safe and environment-friendly, favoring industrial production of polymorphs.

The embodiments of the present disclosure have been described above. However, the present disclosure is not limited to the embodiments described above. Any modification, equivalent, improvement, and the like made without departing from the spirit and principle of the present disclosure shall fall within the protection scope of the present disclosure.

## Claims

1. A salt of compound A, wherein compound A is shown as the following structure: 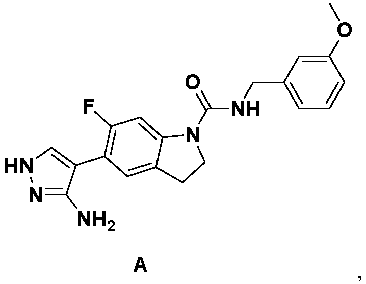 the salt is an acid addition salt of compound A with any one of the following acids: hydrochloric acid, *p*-toluenesulfonic acid, benzenesulfonic acid, maleic acid, tartaric acid, oxalic acid, fumaric acid, sulfuric acid, methanesulfonic acid, phosphoric acid, succinic acid, and citric acid.

2. The salt according to claim 1, wherein the acid is hydrochloric acid, *p*-toluenesulfonic acid, benzenesulfonic acid, maleic acid, tartaric acid, oxalic acid, or fumaric acid;
the acid addition salt is a hydrochloride salt of compound A, a *p*-toluenesulfonate salt of compound A, a benzenesulfonate salt of compound A, a maleate salt of compound A, a tartrate salt of compound A, an oxalate salt of compound A, or a fumarate salt of compound A;
preferably, in the salt of compound A, compound A and the acid are in a molar ratio of 5:1-1:5, e.g., 3:1, 2:1, 1:1, 1:1.5, 1:2, 1:2.5, or 1:3;
preferably, the salt of compound A is in an amorphous or crystal form;
preferably, the hydrochloride salt of compound A is in an amorphous or crystal form of the hydrochloride salt of compound A, the p-toluenesulfonate salt of compound A is in an amorphous or crystal form of the *p*-toluenesulfonate salt of compound A, the benzenesulfonate salt of compound A is in an amorphous or crystal form of the benzenesulfonate salt of compound A, the maleate salt of compound A is in an amorphous or crystal form of the maleate salt of compound A, the tartrate salt of compound A is in an amorphous or crystal form of the tartrate salt of compound A, the oxalate salt of compound A is in an amorphous or crystal form of the oxalate salt of compound A, and the fumarate salt of compound A is in an amorphous or crystal form of the fumarate salt of compound A.

3. The salt according to claim 2, wherein the hydrochloride salt of compound A is in a crystal form, which is named as a hydrochloride crystal form I, wherein the hydrochloride crystal form I has characteristic peaks at 2θ angles of 5.93° ± 0.20°, 14.92° ± 0.20°, and 24.07° ± 0.20° by X-ray powder diffraction using Cu-Kα radiation;
preferably, the hydrochloride crystal form I has characteristic peaks at 2θ angles of 5.93° ± 0.20°, 11.96° ± 0.20°, 14.92° ± 0.20°, 17.98° ± 0.20°, 24.07° ± 0.20°, 26.61° ± 0.20°, and 27.18° ± 0.20° by X-ray powder diffraction using Cu-Kα radiation;
further preferably, the hydrochloride crystal form I has characteristic peaks at 2θ angles of 5.93° ± 0.20°, 11.96° ± 0.20°, 12.56° ± 0.20°, 14.92° ± 0.20°, 17.98° ± 0.20°, 18.96° ± 0.20°, 21.02° ± 0.20°, 24.07° ± 0.20°, 25.53° ± 0.20°, 26.61° ± 0.20°, 27.18° ± 0.20°, and 31.66° ± 0.20° by X-ray powder diffraction using Cu-Kα radiation;
preferably, the hydrochloride crystal form I has characteristic peaks at 2θ angles by X-ray powder diffraction as shown in Table 5', with a tolerance range of ± 0.2°;
preferably, the hydrochloride crystal form I has an XRPD pattern substantially as shown in FIG. 1(a); preferably, in the hydrochloride crystal form I, compound A and the hydrochloric acid are in a molar ratio of 1:1;
preferably, the hydrochloride crystal form I is a hydrate, preferably a monohydrate;
preferably, the *p*-toluenesulfonate salt of compound A is in a crystal form, which is named as a *p-*toluenesulfonate crystal form I, wherein the *p*-toluenesulfonate crystal form I has characteristic peaks at 2θ angles of 7.55° ± 0.20°, 8.61° ± 0.20°, 14.75° ± 0.20°, 15.99° ± 0.20°, and 23.38° ± 0.20° by X-ray powder diffraction using Cu-Kα radiation;
preferably, the *p*-toluenesulfonate crystal form I has characteristic peaks at 2θ angles of 7.55° ± 0.20°, 8.61° ± 0.20°, 14.75° ± 0.20 °, 15.99° ± 0.20°, 19.64° ± 0.20°, 19.91° ± 0.20°, 23.38° ± 0.20°, 24.02° ± 0.20°, and 24.60° ± 0.20° by X-ray powder diffraction using Cu-Kα radiation;
further preferably, the *p*-toluenesulfonate crystal form I has characteristic peaks at 2θ angles of 5.49° ± 0.20°, 7.55° ± 0.20°, 8.61° ± 0.20°, 9.14° ± 0.20°, 10.05° ± 0.20°, 14.39° ± 0.20°, 14.75° ± 0.20°, 15.99° ± 0.20°, 19.64° ± 0.20°, 19.91° ± 0.20°, 20.67° ± 0.20°, 23.38° ± 0.20°, 24.02° ± 0.20°, and 24.60° ± 0.20° by X-ray powder diffraction using Cu-Kα radiation;
preferably, the *p*-toluenesulfonate crystal form I has characteristic peaks at 2θ angles by X-ray powder diffraction as shown in Table 8', with a tolerance range of ± 0.2°;
preferably, the *p*-toluenesulfonate crystal form I has an XRPD pattern substantially as shown in FIG. 4(a);
preferably, in the *p*-toluenesulfonate crystal form I, compound A and the *p*-toluenesulfonic acid are in a molar ratio of 1:1;
preferably, the *p*-toluenesulfonate crystal form I is a hydrate, preferably a monohydrate;
preferably, the benzenesulfonate salt of compound A is in a crystal form, which is named as a benzenesulfonate crystal form I, wherein the benzenesulfonate crystal form I has characteristic peaks at 2θ angles of 7.96° ± 0.20°, 9.00° ± 0.20°, 15.80° ± 0.20°, 20.49° ± 0.20°, and 24.61° ± 0.20° by X-ray powder diffraction using Cu-Kα radiation;
preferably, the benzenesulfonate crystal form I has characteristic peaks at 2θ angles of 7.96° ± 0.20°, 9.00° ± 0.20°, 15.28° ± 0.20°, 15.80° ± 0.20°, 19.97° ± 0.20°, 20.49° ± 0.20°, and 24.61° ± 0.20° by X-ray powder diffraction using Cu-Kα radiation;
further preferably, the benzenesulfonate crystal form I has characteristic peaks at 2θ angles of 7.96° ± 0.20°, 9.00° ± 0.20°, 9.79° ± 0.20°, 10.30° ± 0.20°, 14.48° ± 0.20°, 15.28° ± 0.20°, 15.80° ± 0.20°, 17.09° ± 0.20°, 17.29° ± 0.20°, 19.24° ± 0.20°, 19.97° ± 0.20°, 20.49° ± 0.20°, 23.29° ± 0.20°, 24.61° ± 0.20°, and 25.24° ± 0.20° by X-ray powder diffraction using Cu-Kα radiation;
preferably, the benzenesulfonate crystal form I has characteristic peaks at 2θ angles by X-ray powder diffraction as shown in Table 10', with a tolerance range of ± 0.2°;
preferably, the benzenesulfonate crystal form I has an XRPD pattern substantially as shown in FIG. 7(a);
preferably, in the benzenesulfonate crystal form I, compound A and the benzenesulfonic acid are in a molar ratio of 1:1;
preferably, the benzenesulfonate crystal form I is a hydrate, preferably a monohydrate.

4. The salt according to claim 2, wherein the maleate salt of compound A is in a crystal form, which is named as a maleate crystal form I, wherein the maleate crystal form I has characteristic peaks at 2θ angles of 4.22° ± 0.20°, 7.29° ± 0.20°, 16.13° ± 0.20°, 17.19° ± 0.20°, and 26.07° ± 0.20° by X-ray powder diffraction using Cu-Kα radiation;
preferably, the maleate crystal form I has characteristic peaks at 2θ angles of 4.22° ± 0.20°, 7.29° ± 0.20°, 12.25° ± 0.20°, 14.68° ± 0.20°, 15.34° ± 0.20°, 16.13° ± 0.20°, 17.19° ± 0.20°, 19.21° ± 0.20°, 22.59° ± 0.20°, 26.07° ± 0.20° by X-ray powder diffraction using Cu-Kα radiation;
preferably, the maleate crystal form I has characteristic peaks at 2θ angles by X-ray powder diffraction as shown in Table 12', with a tolerance range of ± 0.2°;
preferably, the maleate crystal form I has an XRPD pattern substantially as shown in FIG. 10(a);
preferably, in the maleate crystal form I, compound A and the maleic acid are in a molar ratio of 1:1;
preferably, the maleate crystal form I is a solvate, and more preferably, the maleate crystal form I is an ethyl acetate solvate;
preferably, the maleate salt of compound A is in a crystal form, which is named as a maleate crystal form II, wherein the maleate crystal form II has characteristic peaks at 2θ angles of 7.99° ± 0.20° and 20.17° ± 0.20° by X-ray powder diffraction using Cu-Kα radiation;
preferably, the maleate crystal form II has characteristic peaks at 2θ angles of 3.96° ± 0.20°, 7.99° ± 0.20°, 20.17° ± 0.20°, 24.23° ± 0.20°, and 28.31° ± 0.20° by X-ray powder diffraction using Cu-Kα radiation;
further preferably, the maleate crystal form II has characteristic peaks at 2θ angles of 3.96° ± 0.20°, 7.99° ± 0.20°, 9.25° ± 0.20°, 11.19° ± 0.20°, 13.25° ± 0.20°, 20.17° ± 0.20°, 23.85° ± 0.20°, 24.23° ± 0.20°, 27.47° ± 0.20°, and 28.31° ± 0.20° by X-ray powder diffraction using Cu-Kα radiation;
preferably, the maleate crystal form II has characteristic peaks at 2θ angles by X-ray powder diffraction as shown in Table 12", with a tolerance range of ± 0.2°;
preferably, the maleate crystal form II has an XRPD pattern substantially as shown in FIG. 11(a);
preferably, in the maleate crystal form II, compound A and the maleic acid are in a molar ratio of 1:1;
preferably, the maleate crystal form II is a solvate and/or hydrate, and more preferably, the maleate crystal form II is a methyl tert-butyl ether (MTBE) solvate and/or hydrate;
preferably, the oxalate salt of compound A is in a crystal form, which is named as an oxalate crystal form I, wherein the oxalate crystal form I has characteristic peaks at 2θ angles of 5.26° ± 0.20°, 12.24° ± 0.20°, and 25.75° ± 0.20° by X-ray powder diffraction using Cu-Kα radiation;
preferably, the oxalate crystal form I has characteristic peaks at 2θ angles of 4.94° ± 0.20°, 5.26° ± 0.20°, 7.25° ± 0.20°, 12.24° ± 0.20°, 14.77° ± 0.20°, 16.55° ± 0.20°, 20.95° ± 0.20°, and 25.75° ± 0.20° by X-ray powder diffraction using Cu-Kα radiation;
further preferably, the oxalate crystal form I has characteristic peaks at 2θ angles of 4.94° ± 0.20°, 5.26° ± 0.20°, 7.25° ± 0.20°, 12.24° ± 0.20°, 14.17° ± 0.20°, 14.77° ± 0.20°, 16.03° ± 0.20°, 16.55° ± 0.20°, 20.21° ± 0.20°, 20.95° ± 0.20°, 25.75° ± 0.20°, and 30.87° ± 0.20° by X-ray powder diffraction using Cu-Kα radiation;
preferably, the oxalate crystal form I has characteristic peaks at 2θ angles by X-ray powder diffraction as shown in Table 14', with a tolerance range of ± 0.2°;
preferably, the oxalate crystal form I has an XRPD pattern substantially as shown in FIG. 15(a);
preferably, in the oxalate crystal form I, compound A and the maleic acid are in a molar ratio of 1:1;
preferably, the oxalate crystal form I is an anhydrate.

5. The salt according to claim 2, wherein the fumarate salt of compound A comprises a salt formed from compound A and the fumaric acid according to a molar ratio of 1:1 or 2:1;
preferably, the fumarate salt of compound A is in a crystal form, which is named as a fumarate crystal form I, wherein the fumarate crystal form I has characteristic peaks at 2θ angles of 3.90° ± 0.20°, 13.93° ± 0.20°, 16.86° ± 0.20°, and 26.37° ± 0.20° by X-ray powder diffraction using Cu-Kα radiation;
preferably, the fumarate crystal form I has characteristic peaks at 2θ angles of 3.90° ± 0.20°, 10.45° ± 0.20°, 13.93° ± 0.20°, 16.86° ± 0.20°, 17.73° ± 0.20°, 21.39° ± 0.20°, 23.68° ± 0.20°, 26.37° ± 0.20°, 27.40° ± 0.20°, and 27.87° ± 0.20° by X-ray powder diffraction using Cu-Kα radiation;
preferably, the fumarate crystal form I has characteristic peaks at 2θ angles by X-ray powder diffraction as shown in Table 19', with a tolerance range of ± 0.2°;
preferably, the fumarate crystal form I has an XRPD pattern substantially as shown in FIG. 21(a); preferably, in the fumarate crystal form I, compound A and the fumaric acid are in a molar ratio of 2:1;
preferably, the fumarate crystal form I is an anhydrate;
preferably, the fumarate salt of compound A is in a crystal form, which is named as a fumarate crystal form II, wherein the fumarate crystal form II has characteristic peaks at 2θ angles of 22.06° ± 0.20° and 25.20° ± 0.20° by X-ray powder diffraction using Cu-Kα radiation;
preferably, the fumarate crystal form II has characteristic peaks at 2θ angles of 22.06° ± 0.20°, 22.50° ± 0.20°, 25.20° ± 0.20°, and 27.54° ± 0.20° by X-ray powder diffraction using Cu-Kα radiation;
further preferably, the fumarate crystal form II has characteristic peaks at 2θ angles of 11.44° ± 0.20°, 13.74° ± 0.20°, 22.06° ± 0.20°, 22.50° ± 0.20°, 24.60° ± 0.20°, 25.20° ± 0.20°, 27.54° ± 0.20°, and 28.78° ± 0.20° by X-ray powder diffraction using Cu-Kα radiation;
preferably, the fumarate crystal form II has characteristic peaks at 2θ angles by X-ray powder diffraction as shown in Table 20', with a tolerance range of ± 0.2°;
preferably, the fumarate crystal form II has an XRPD pattern substantially as shown in FIG. 24(a);
preferably, in the fumarate crystal form II, compound A and the fumaric acid are in a molar ratio of 2:1;
preferably, the fumarate crystal form II is an anhydrate;
preferably, the tartrate salt of compound A is in a crystal form, which is named as a tartrate crystal form I, wherein the tartrate crystal form I has characteristic peaks at 2θ angles of 16.98° ± 0.20°, 17.85° ± 0.20°, 19.66° ± 0.20°, and 25.58° ± 0.20° by X-ray powder diffraction using Cu-Kα radiation;
preferably, the tartrate crystal form I has characteristic peaks at 2θ angles of 13.38° ± 0.20°, 16.98° ± 0.20°, 17.85° ± 0.20°, 18.53° ± 0.20°, 19.66° ± 0.20°, 25.58° ± 0.20°, and 26.72° ± 0.20° by X-ray powder diffraction using Cu-Kα radiation;
preferably, the tartrate crystal form I has characteristic peaks at 2θ angles of 13.38° ± 0.20°, 16.98° ± 0.20°, 17.27° ± 0.20°, 17.85° ± 0.20°, 18.53° ± 0.20°, 19.66° ± 0.20°, 20.46° ± 0.20°, 22.86° ± 0.20°, 25.58° ± 0.20°, 26.08° ± 0.20°, and 26.72° ± 0.20° by X-ray powder diffraction using Cu-Kα radiation;
preferably, the tartrate crystal form I has characteristic peaks at 2θ angles by X-ray powder diffraction as shown in Table 22', with a tolerance range of ± 0.2°;
preferably, the tartrate crystal form I has an XRPD pattern substantially as shown in FIG. 33(a);
preferably, in the tartrate crystal form I, compound A and the tartaric acid are in a molar ratio of 1:1;
preferably, the tartrate crystal form I is an anhydrate.

6. A preparation method for the salt of compound A according to any one of claims 1-5, comprising forming a salt of compound A with an acid, wherein the acid is selected from hydrochloric acid, *p-*toluenesulfonic acid, benzenesulfonic acid, maleic acid, tartaric acid, oxalic acid, fumaric acid, sulfuric acid, methanesulfonic acid, phosphoric acid, succinic acid, and citric acid, and preferably is hydrochloric acid, *p*-toluenesulfonic acid, benzenesulfonic acid, maleic acid, tartaric acid, oxalic acid, or fumaric acid.

7. The preparation method according to claim 6, comprising the following steps: subjecting compound A to a salt-forming reaction with the acid in a solvent, stirring the mixture until a solid is precipitated, and drying the solid to obtain the salt;
if no solid is precipitated by stirring, adding an anti-solvent to the system, and drying a solid after the solid is precipitated to obtain the salt; wherein
preferably, the solvent is selected from one, two or more of EA (ethyl acetate), 2-Me-THF (2-methyl-tetrahydrofuran), ACN (acetonitrile), DCM (dichloromethane), EtOH (ethanol), MeOH (methanol), IPA (isopropyl alcohol), THF (tetrahydrofuran), and IPAc (isopropyl acetate), or a mixed solvent of any one, two or more of the solvents described above with MTBE (methyl *tert*-butyl ether);
preferably, the anti-solvent is selected from MTBE (methyl *tert*-butyl ether) and/or ACN (acetonitrile).

8. A pharmaceutical composition comprising the salt according to any one of claims 1-5, wherein preferably, the pharmaceutical composition further comprises a pharmaceutically acceptable carrier; preferably, the pharmaceutical composition further comprises a second active ingredient, wherein, for example, the second active ingredient is one, two or more of other ROCK inhibitors, tyrosine kinase inhibitors, tyrosinase inhibitors, inhibitors of profibrotic cytokines, serum amyloid P inhibitors, autotaxin-lysophosphatidic acid pathway inhibitors, GPR40 agonists, GPR84 antagonists, anti-acid drugs, and antibiotics.

9. Use of the salt according to any one of claims 1-5 or the pharmaceutical composition according to claim 8 for manufacturing a preparation.

10. The use according to claim 9, wherein the preparation is a ROCK antagonist; preferably, the ROCK antagonist is for use in the prevention and/or treatment of one or more diseases caused by high expression or excessive activation of ROCK;
preferably, the disease is selected from cardiovascular and cerebrovascular diseases, neurological diseases, fibrotic diseases, ocular diseases, tumors, arterial thrombotic disorders, radiation damage, respiratory diseases, metabolic diseases, and autoimmune diseases;
preferably, the disease includes atherosclerosis, acute coronary syndrome, hypertension, cerebral vasospasm, cerebral ischemia, ischemic stroke, restenosis, heart disease, heart failure, cardiac hypertrophy, myocardial ischemia-reperfusion injury, diabetes, diabetic nephropathy, cancer, neuronal degeneration, nerve injury diseases, spinal cord injury, erectile dysfunction, platelet aggregation, leukocyte aggregation, glaucoma, ocular hypertension, asthma, osteoporosis, pulmonary fibrosis (such as idiopathic pulmonary fibrosis), hepatic fibrosis, renal fibrosis, COPD, kidney dialysis, glomerulosclerosis, fatty liver disease, fatty liver hepatitis, or neuronal degeneration inflammation.

11. A preparation comprising the salt according to any one of claims 1-5, wherein
preferably, the preparation further comprises one or more pharmaceutically acceptable carriers; preferably, the preparation comprises the pharmaceutical composition according to claim 8.

12. The preparation according to claim 11, wherein the preparation is in the form of powders, tablets (such as coated tablets, and sustained-release or controlled-release tablets), lozenges, capsules (such as soft capsules or hard capsules), granules, pills, dispersible powders, suspensions, solutions, emulsions, elixirs, syrups, aerosols, creams, ointments, gels, injections, lyophilized powder injections, suppositories, or the like;
preferably, the preparation is a ROCK antagonist; preferably, the ROCK antagonist is for use in the prevention and/or treatment of one or more diseases caused by high expression or excessive activation of ROCK.
